(19) Europäisches Patentamt
European Patent Office
Office européen des brevets

(11) EP 2 834 240 B1

(12) EUROPÄISCHE PATENTSCHRIFT

(45) Veröffentlichungstag und Bekanntmachung des Hinweises auf die Patenterteilung:
**07.06.2017 Patentblatt 2017/23**

(21) Anmeldenummer: **13717459.5**

(22) Anmeldetag: **05.04.2013**

(51) Int Cl.:
***C07D 471/14*** (2006.01) ***A61K 31/4745*** (2006.01)
***A61P 35/00*** (2006.01) ***A61P 29/00*** (2006.01)
***A61P 31/00*** (2006.01) ***A61P 31/04*** (2006.01)
***A61P 31/10*** (2006.01) ***A61P 31/12*** (2006.01)
***A61P 33/00*** (2006.01)

(86) Internationale Anmeldenummer:
**PCT/EP2013/057212**

(87) Internationale Veröffentlichungsnummer:
**WO 2013/150140 (10.10.2013 Gazette 2013/41)**

(54) **NEUE PYRIDO[3,4-C][1,9]PHENANTHROLIN- UND 11,12-DIHYDROPYRIDO[3,4-C][1,9]PHENANTHROLIN- DERIVATE UND IHRE VERWENDUNG, INSBESONDERE, ZUR BEHANDLUNG VON KREBS**

NOVEL PYRIDO[3,4-C][1,9]PHENANTHROLINE- AND 11,12-DIHYDROPYRIDO[3,4-C][1,9]PHENANTHROLINE- DERIVATIVES AND THEIR USE, IN PARTICULAR, FOR THE TREATMENT OF CANCER

NOUVEAUX DÉRIVÉS DE PYRIDO[3,4-C][1,9]PHENANTHROLINE ET 11,12-DIHYDROPYRIDO[3,4-C][1,9]PHENANTHROLINE ET LEUR UTILISATION, EN PARTICULIER, POUR LE TRAITEMENT DU CANCER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.04.2012 DE 102012006903**

(43) Veröffentlichungstag der Anmeldung:
**11.02.2015 Patentblatt 2015/07**

(73) Patentinhaber: **Christian-Albrechts-Universität zu Kiel**
**24118 Kiel (DE)**

(72) Erfinder:
- **CLEMENT, Bernd**
**24106 Kiel (DE)**
- **MEIER, Christopher**
**24118 Kiel (DE)**
- **HEBER, Dieter**
**24113 Molfsee (DE)**
- **STENZEL, Lars**
**28876 Oyten (DE)**

(74) Vertreter: **Steinecke, Peter**
**Müller Fottner Steinecke**
**Rechtsanwalts- und Patentanwaltspartnerschaft mbB**
**Postfach 31 01 40**
**80102 München (DE)**

(56) Entgegenhaltungen:
**WO-A2-97/14683**

- **Lars Stenzel: "Synthese neuer Benzo[c]phenanthridin-Derivate und deren Stickstoff-Analoga als potentielle Zytostatika", 9. November 2009 (2009-11-09), Dissertation zur Erlangung des Doktorgrades der Mathematisch-Naturwissenschaftlichen Fakultät der Christian-Albrechts- Universität zu Kiel, XP002697378, Seite 52; Verbindung 82 das ganze Dokument; im besonderen, die Seiten 51-56, das Kapitel 2.3**
- **KOCK, ILKA ET AL: "Synthesis and biological evaluation of 11-substituted 6-aminobenzo[c]phenanthridine derivatives, a new class of antitumor agents", JOURNAL OF MEDICINAL CHEMISTRY , 48(8), 2772-2777 CODEN: JMCMAR; ISSN: 0022-2623, Bd. 48, Nr. 8, 2005, XP002697379,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- MEIER, CHRISTOPHER ET AL: "Synthesis and physicochemical characterization of novel 6-aminopyrido[3,4-c][1,9]phenanthrolines as aza-analogs of benzo[c]phenanthridines", TETRAHEDRON, CODEN: TETRAB; ISSN: 0040-4020, Bd. 68, Nr. 44, 28. August 2012 (2012-08-28), Seiten 9105-9112, XP002697380,

## Beschreibung

Die vorliegende Erfindung betrifft neue aromatische Heterocyclen, ein Verfahren zu deren Herstellung sowie Arzneimittel, enthaltend diese Heterocyclen.

Viele Krebserkrankungen sind bislang nicht durch selektiv wirkende Wirkstoffe therapierbar. Nach Angaben der World Health Organization (WHO) wurde weltweit im Jahr 2000 bei ca. 10 Millionen Menschen Krebs diagnostiziert, ca. 6 Millionen verstarben (World Cancer Report 2003, www.iarc.fr/IARCPress/pdfs/wcr/WorldCancerReport.pdf). Nach Schätzung der WHO wird die Zahl der durch Krebserkrankungen hervorgerufenen Todesfälle bis 2030 auf ca. 11,5 Millionen pro Jahr ansteigen (Worlds Health Statistics - 2007, www.who.int/whosis/whostat2007_10-highlights.pdf). Im Hinblick auf die große Anzahl von Menschen, die an Krebs erkrankt sind, die ungünstige Prognose für die Heilung bestimmter Krebsarten aufgrund der schlechten Wirksamkeit bisheriger Medikamente, Nebenwirkungen und Resistenzentwicklung gegenüber eingesetzten Arzneimitteln besteht ein dringender Bedarf an neuen Krebsmedikamenten.

Aus der DE 195 38 088 A1 sind 6-Aminobenzo[c]phenanthridine bekannt, welche teilweise über antitumorale, antimikrobielle, antifungizide, antivirale und antiinflammatorische Eigenschaften verfügen. Die genannten Verbindungen weisen aber noch Nachteile im pharmakologischen Profil insbesondere der geringen Wasserlöslichkeit auf.

Es ist daher Aufgabe der Erfindung neue Arzneimittel zur Verfügung zu stellen. Des Weiteren ist es Aufgabe der Erfindung neue Arzneimittel insbesondere zur Therapie gegen Krebserkrankungen zur Verfügung zu stellen. Eine weitere Aufgabe der Erfindung ist es neue Arzneimittel mit einer pharmazeutisch akzeptablen Wasserlöslichkeit zur Verfügung zu stellen sowie ein Herstellungsverfahren wie nachstehend beschrieben, durch die die erfindungsmäßen Verbindungen erhalten werden können.

Die Aufgabe wird durch die in den Ansprüchen und der vorliegenden Beschreibung gekennzeichneten Ausführungsformen gelöst. Die Unteransprüche und Beispiele geben vorteilhafte Ausgestaltungen der Erfindung an.

In einem Aspekt betrifft die vorliegende Erfindung die Bereitstellung von Phenanthrolinderivate der allgemeinen Formeln I und II

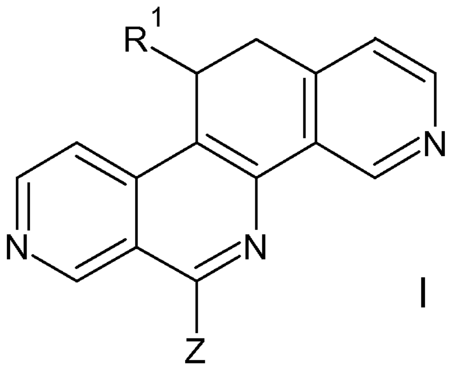

I

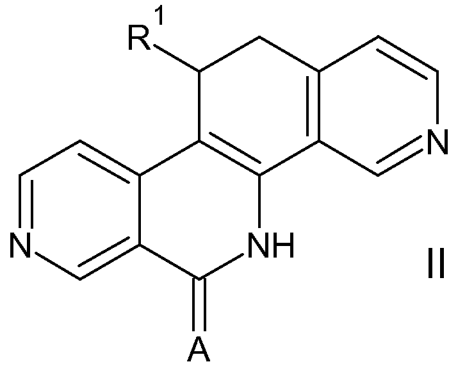

II

wobei $R^1$ gleich einem Phenylring der Formel

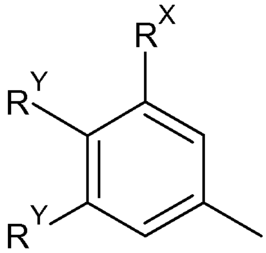

ist, der mindestens einen Substituenten $R^X$ ausgewählt aus der Gruppe Cl, Br oder I in meta-Stellung trägt, wobei $R^Y$, welches gleich oder verschieden sein kann, gleich H, OH, OMe, OEt oder Halogen ist; sowie der allgemeinen Formeln III und IV,

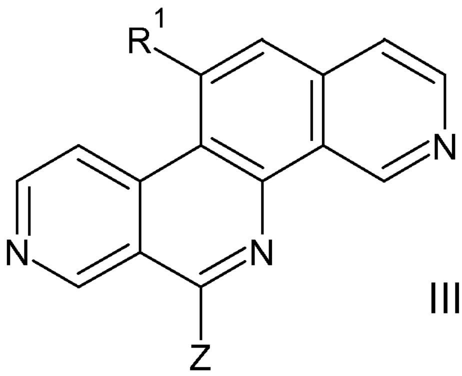

III

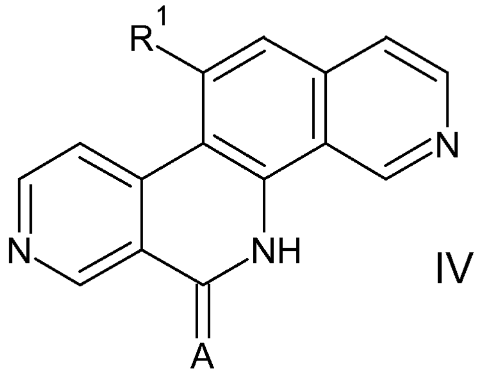

IV

wobei $R^1$ entweder gleich einem Phenylring der Formel

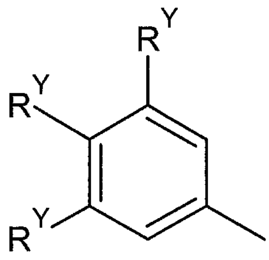

ist, der mindestens einen Substituenten $R^Y$ in meta-Stellung trägt, wobei $R^Y$, welches gleich oder verschieden sein kann, gleich H, OH, OMe, OEt oder Halogen ist oder gleich einem fünfgliedrigen aromatischen Heterocyclus der Formel

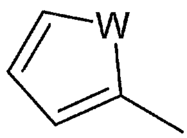

ist,

wobei W gleich O, S oder NH ist; und
wobei Z gleich H, F, Cl, Br, I, NHX, OX, SX,
wobei X gleich einem H, Dimethylaminoalkyl-, Diethylaminoalkyl-, ω-(1,3-diazol-1-yl)-alkyl-, Hydroxyalkyl-, Alkoxyalkyl-, Thioalkyl-, Alkylthioalkylgruppe ist,
wobei Alkyl gleich Methyl, Ethyl oder Propyl
und wobei A gleich O oder S ist,
sowie deren pharmazeutisch verträgliche Salze und Solvate.

In einem weiteren Aspekt betrifft die Erfindung ein Verfahren zur Herstellung einer Verbindung gemäß der vorliegenden Erfindung wie vorstehend beschrieben, umfassend mindestens die folgenden Schritte:

(i) Umsetzung von substituierten Aldehyden der allgemeinen Formel $R^1$ - CHO (V) mit substituierten 4-Methylpyridin-3-carbonitrilen der allgemeinen Formel

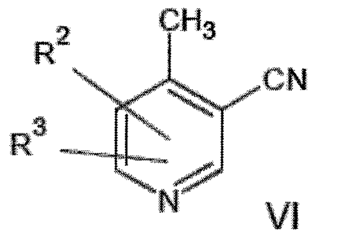

VI

in aprotischen bipolaren Lösungsmitteln in Gegenwart von Basen zu einer Verbindung der allgemeinen Formel

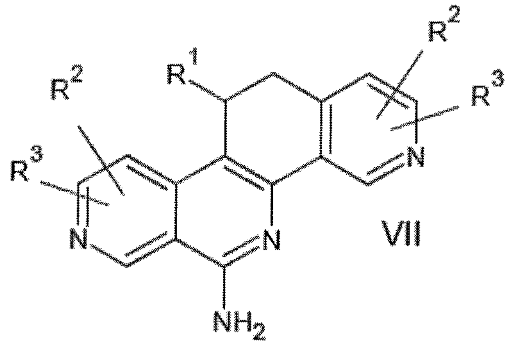

VII

(i) Isolierung der Produkte und Derivatisierung an Position 6 unter Einfügen der Reste $R^4$ zu den Derivaten mit A- oder Z-Substitution in 6-Position, gemäß der Formeln I bis IV,

wobei $R^1$ ein H, ein cyclischer oder acyclischer, verzweigter oder unverzweigter aliphatischer Kohlenwasserstoffrest, einfach oder mehrfach substituiert, ein aromatischer carbocyclischer oder heterocyclischer Rest, einfach oder mehrfach substituiert ist, und

$R^2$ und $R^3$, gleich oder verschieden sein können und ein H, Alkyloxyrest, Alkylenoxyrest, Halogenatom oder eine Nitrogruppe ist, und

$R^4$ ein H, eine Monoamino-, Alkylamino-, Dialkylamino-, (Dialkyl-)aminoalkylamin-, Alkyl-, Alkoxy-, (Dialkyl-)aminoalkyloxy-, Hydroxy-, Hydroxyalkylamino-, Hydroxyalkyloxy-, Thiol-, (Dialkyl-) aminoalkylthio-, Thioalkyl-, Alkylthioalkylgruppe oder ein Halogenatom ist.

Die anschließende Derivatisierung an Position 6 unter Einfügen der Reste $R^4$ zu den Derivaten mit A - oder Z-Substitution in 6-Position entsprechend Verfahrensschritt (ii) führt zu den erfindungsgemäßen Verbindungen wie in der allgemeinen Formel III oder IV dargestellt.

Daher betrifft die vorliegende Erfindung ein Verfahren zur Herstellung einer Verbindung wie vorstehend beschrieben, wobei zwischen den beiden Schritten (i) und (ii) optional der Verfahrensschritt

(i.a.) Dehydrierung und Isolierung der hieraus resultierenden Produkte der allgemeinen Formel

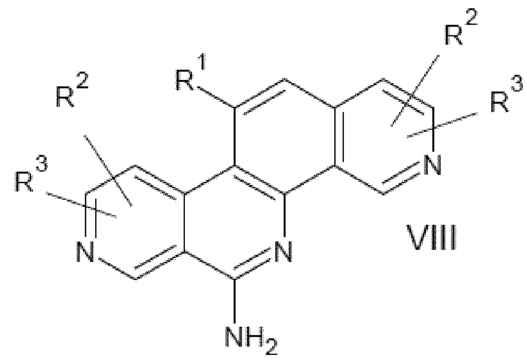

VIII

eingeführt sein kann.

Insbesondere betrifft die vorliegende Erfindung ein wie oben beschriebenes Verfahren, wobei das aprotische dipolare Lösungsmittel vorzugsweise ein Amid wie Dimethylformamid, Dimethylacetamid, Diethylacetamid, Hexamethylphosphorsäuretrisamid oder ein Harnstoff wie Tetramethylharnstoff, 1,3-Dimethyltetrahydropyrimidin-2-on und 1,3-Dimethylimidazolidinon oder ein Dimethylsulfoxid ist und wobei die Base ein Alkali- oder Erdalkalihydrid wie Natriumhydrid, Alkaliamid wie Natriumamid, Natriummethylacetamid, Alkali-, Erdalkali- oder Aluminiumalkoholat wie Kaliumtert-butylat, Natriummethylat, Natriumethylat oder Aluminiumethylat ist.

Es hat sich in den erfindungsgemäß durchgeführten Experimenten gezeigt, dass es überraschenderweise möglich ist, die erfindungsgemäßen Phenanthrolinderivate durch einfache Umsetzung von entsprechend substituierten Aldehyden mit entsprechend substituierten 4-Methylpyridin-3-carbonitrilen zu erhalten.

Der Reaktionsablauf lässt sich wie folgt darstellen:

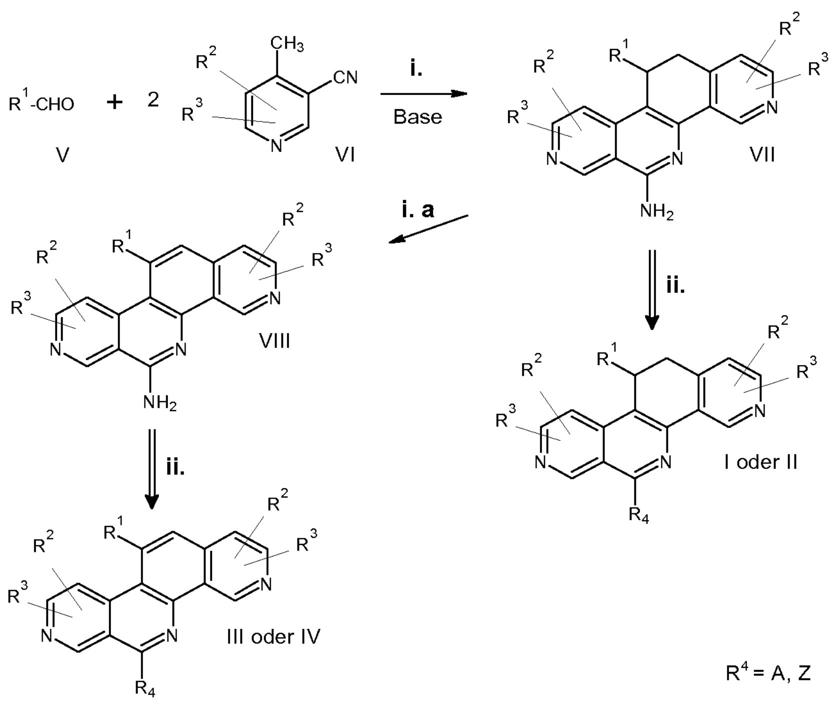


V, VI, VII, VIII, I oder II, III oder IV

$R^4$ = A, Z

Im Einzelnen wird dabei so vorgegangen, dass eine Umsetzung von einem Aldehyd der Formel

$R_1$-CHO V,

worin $R^1$ sowohl Wasserstoff, ein cyclischer oder acyclischer, verzweigter oder unverzweigter aliphatischer Kohlenwasserstoffrest, der einfach oder mehrfach substituiert sein kann, als auch ein aromatischer carbocyclischer oder heterocyclischer Rest, der einfach oder mehrfach substituiert sein kann, mit 2 Mol eines 4-Methylpyridin-3-carbonitrils der Formel VI,

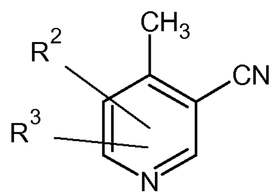

VI

wobei $R^2$ und $R^3$, die gleich oder verschieden sein können, ein H, einen Alkyloxyrest, einen Alkylenoxyrest, ein Halogenatom oder eine Nitrogruppe bedeuten, in Gegenwart von Base in einem aprotischen dipolaren Lösungsmittel zu einem 6-Amino-11,12-dihydropyrido[3,4-*c*][1,9]phenanthrolin der Formel VII erfolgt.

Nach Isolierung durch Ausfällen der freien Base, Filtration und anschließender Aufreinigung, beispielsweise Umkristallisation (Organikum, 21. Auflage, 2001, Wiley-VCH) können diese Derivate der Formel VII mit den für die jeweiligen Reste $R^4$ an Position 6 entsprechenden chemischen Methoden (Diazotierung, Umsetzung von Carbonsäurederivaten mit anorganischen Säurechloriden, nukleophile Substitution am Aromaten, Synthese von Thionen/Thiolen und Reduktionsreaktionen, wie z.B. Entschwefelungen), welche dem Fachmann beispielsweise aus Organikum, 21. Auflage, 2001, Wiley-VCH oder Organische Chemie, K. Peter C. Vollhardt, 3. Auflage, 2000 bekannt sind, in einem oder mehreren Schritten zu den Derivaten mit der Formel I oder II umgesetzt werden oder es kann nach dem Fachmann allgemein bekannten Methoden (z.B. aus "Comprehensive Organic Transformations", Richard C. Larock, 1989) mit einem geeigneten Dehydrierungsmittel in Ab- oder Anwesenheit von Lösungsmittel eine Dehydrierung zu den entsprechenden 6-Aminopyrido[3,4-*c*][1,9]phenanthrolinen der Formel VIII erfolgen, welche im Anschluss nach Isolierung mit den für die jeweiligen Reste $R^4$ an Position 6 entsprechenden, dem Fachmann bekannten chemischen Methoden (Organikum, 21. Auflage, 2001, Wiley-VCH; Organische Chemie, K. Peter C. Vollhardt, 3. Auflage, 2000), in einem oder mehreren Schritten zu den Derivaten mit der Formel III oder IV umgesetzt werden können, wobei der Rest $R^4$ ein Wasserstoffatom, eine Monoaminogruppe, eine Alkylaminogruppe, eine Dialkylaminogruppe, eine (Dialkyl-)aminoalkylamingruppe, eine Alkylgruppe, eine Alkoxygruppe, eine (Dialkyl-)aminoalkyloxygruppe, eine Hydroxygruppe, eine Hydroxyalkylaminogruppe, eine Hydroxyalkyloxygruppe, eine Thiolgruppe, eine (Dialkyl-) aminoalkylthiogruppe eine Thiolalkylaminogruppe, eine Thiolalkylthiogruppe, sowie ein Halogenatom sein kann. Eine Auswahl an N-substituierten Alkylendiamin-Seitenketten an Position 6 ist z.B. bei *Genes* dargestellt (Genes et al., Eu. J. Med. Chem. (46), 2011, 2117-2131). Die Einfuhr von Sauerstoff-, Schwefel- und Kohlenstoffnukleophilen wird z.B. von *Cherng* beschrieben (Cherng, Tetrahedron 58, 2002, 4931-4935), des Weiteren gelten die allgemein gültigen Methoden der nukleophilen Substitution am Aromaten, dem Fachmann bekannt aus Organikum, 21. Auflage, 2001, Wiley-VCH oder Organische Chemie, K. Peter C. Vollhardt, 3. Auflage, 2000.

Als aprotische dipolare Lösungsmittel für die erfindungsgemäße Reaktion zur Herstellung von entsprechend substituierten 6-Amino-11,12-dihydropyrido[3,4-*c*][1,9]phenanthrolinen der Formel VII sind vorzugsweise Amide wie Dimethylformamid, Dimethylacetamid, Diethylacetamid, Hexamethylphosphorsäure-trisamid oder Harnstoffe wie Tetramethylharnstoff, 1,3-Dimethyltetrahydro-pyrimidin-2-on und 1,3-Dimethylimidazolidinon oder Dimethylsulfoxid verwendbar.

Als Base können beispielsweise eingesetzt werden:

Alkali- oder Erdalkalihydride, wie Natriumhydrid, Alkaliamide wie Natriumamid, Natriummethylacetamid, Alkali-, Erdalkali- oder Aluminiumalkoholate wie Kalium-tert.-butylat, Natriummethylat, Natriumethylat oder Aluminiumethylat.

Die Reaktion kann wie folgt durchgeführt werden: Zu einer Lösung der Base in einem geeigneten dipolaren Lösungsmittel wird unter Inertbegasung eine Lösung der Verbindung V und VI in demselben Lösungsmittel langsam zugetropft. Nach mehrstündigem Rühren bei 25 - 40 °C unter Inertbegasung wird der Ansatz auf Eiswasser gegossen und der Rückstand abfiltriert. Die abfiltrierte Lösung wird mit einem geeigneten organischen Lösungsmittel ausgeschüttelt. Die organische Phase wird weitgehend eingeengt und anschließend im Vakuum destilliert. Die vereinigten Rückstände können durch Umkristallisation in einem geeigneten Lösungsmittel aufgereinigt werden. Das 6-Amino-11,12-dihydropyrido[3,4-*c*][1,9]-phenanthrolin VII kann dann nach allgemein gültigen Methoden (Comprehensive Organic Transformations, Richard C. Larock, 1989) mit einem geeigneten Dehydrierungsmittel in Gegenwart oder Abwesenheit eines inerten Lösungsmittels zum 6-Amino-pyrido[3,4-*c*][1,9]phenanthrolin VIII dehydriert werden. Die Aminogruppe in 6-Position kann in einem oder mehreren Schritten mit allgemein gültigen chemischen Methoden (z.B Organikum, 21. Auflage, 2001, Wiley-VCH; Organische Chemie, K. Peter C. Vollhardt, 3. Auflage, 2000) gegen die oben genannten genannten Reste $R^4$ ausgetauscht werden, so dass man zu den Formeln I oder II, bzw. für die 11,12-dehydrierten Derivate zu den Formeln III oder IV gelangt.

Besonders hervorzuheben beim erfindungsgemäßen Verfahren ist, dass sich hiermit Phenanthrolinderivate synthetisieren lassen, die in 11-Stellung sowohl aliphatische, als auch substituierte oder unsubstituierte aromatische Reste aufweisen. Es ist überraschend, dass die Synthese durch die vorstehend beschriebene einfache Reaktion möglich ist, wobei aufgrund der einzusetzenden Ausgangssubstanz Aldehyd eine sehr große Variationsbreite in Bezug auf die zu erhaltenden Produkte besteht. Auch an der 6-Position erhält man eine beachtliche Variabilität durch die Möglichkeit des Austauschs der Aminogruppe gegen die genannten Reste. Durch die Kombination der beiden variablen Positionen ergibt sich ein sehr großes Spektrum an Verbindungen und die Möglichkeit des Aufbaus einer großen Bibliothek potentiell zytostatisch wirksamer Substanzen.

Es werden auch Verbindungen, welche durch ein Verfahren wie vorstehend beschrieben erhältlich sind, offenbart.

In einem weiteren Teilaspekt betrifft die Erfindung die vorstehenden Verbindungen zur Verwendung als Arzneimittel, insbesondere zur Verwendung in der Krebstherapie.

Bevorzugt ist eine erfindungsgemäße Verbindung wie oben beschreiben zur Verwendung bei der Behandlung einer Erkrankung ausgewählt aus einer Gruppe bestehend aus mikrobiellen, fungiziden, viralen und/oder inflammatorischen Erkrankungen.

Ferner betrifft die Erfindung in einem Teilaspekt eine erfindungsmäßige Verbindung wie vorstehend beschrieben zur Verwendung bei der Behandlung von Krebs.

Bevorzugt ist der Krebs ausgewählt ist aus einer Gruppe bestehend aus Leukämie, Melanom oder Mammakarzinom.

Des Weiteren betrifft die vorliegende Erfindung eine pharmazeutische Zusammensetzung enthaltend mindestens eine der vorstehend beschriebenen Verbindung in Kombination mit einem geeigneten Hilfsstoff.

Es hat sich in den erfindungsgemäß durchgeführten Experimenten gezeigt, dass die vorstehend beschriebenen Phenanthrolinderivate antitwnorale Eigenschaften besitzen. Aufgrund ihrer strukturellen Ähnlichkeit ist anzunehmen, dass sie wie die Benzo[*c*]phenanthridine zudem antimikrobielle, antifungizide, antivirale und antiinflammatorische Eigenschaften besitzen. Zur Untersuchung der pharmakologischen Eigenschaften wurden die Verbindungen der allgemeinen Formeln I und II, sowie Verbindungen der Substanzklasse III in einem "*in-vitro-Antitumor-Screening*" des National Cancer Institute (NCI), Bethesda, Maryland, USA, untersucht. Getestet wurde gegen 60 verschiedene humanpathogene Tumorzellreihen, die neun Krebsarten entstammten (Leukämie, nichtkleinzelliges Lungenkarzinom, Dickdarmkrebs, ZNS-Krebs, Melanom, Eierstockkrebs, Nierenkrebs, Prostatakrebs, Brustkrebs). Zur Ermittlung der Wirksamkeit werden die Tumorzellen den Verbindungen über zwei Tage ausgesetzt und danach indirekt über die Bestimmung der Proteinbiomasse mit Sulforhodamin B (SRB) die Wachstumshemmung ermittelt. Unbehandelte Kulturen dienen als Referenz.

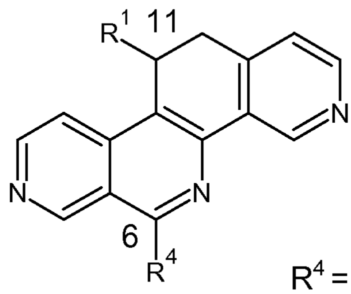

$R^4$ = A, Z

Strukturformel der am National Cancer Institute (NCI) getesteten Verbindungen der erfindungsgemäßen Phenanthrolinderivate der Formeln I und II.

Bei den erfindungsgemäßen Untersuchungen wurden die Derivate zunächst in einer Konzentration von 1 μM an den 60 Zelllinien getestet. Es konnten für mehrere Derivate Wachstumshemmungen festgestellt werden. Überraschenderweise wiesen die erfindungsgemäßen Verbindungen Aktivitäten auf, die außerhalb der Kategorie von in ähnlicher Weise untersuchten Antitumorverbindungen liegen, so dass hier ein ganz neues Wirkungsspektrum erzielt wird.

*Tabelle I* zeigt die Ergebnisse dieses Tests, ausgewählt für 7 verschiedene Derivate der erfindungsgemäßen Phenanthrolinderivate der Formeln I und II mit unterschiedlichen Resten in 11-Position sowie in 6-Position. Die jeweiligen Reste sind in den ersten beiden Zeilen dargestellt. Die linke Spalte zeigt die verwendeten Zelllinien aus 3 ausgewählten Tumorarten, Leukämie, Melanom, sowie Brustkrebs. Die Tabelle macht Aussagen über wachstumshemmende Wirkungen der entsprechenden Derivate mit ihren unterschiedlichen Resten.

| Rest in 11-Position | 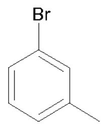 | 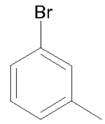 | 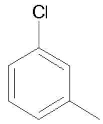 | 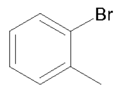 | 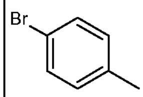 | 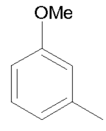 | 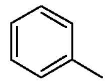 |
|---|---|---|---|---|---|---|---|
| Rest in 6-Position | $NH_2$ | OH | $NH_2$ | $NH_2$ | $NH_2$ | $NH_2$ | $NH_2$ |
| **Zelllinien** | **Zellwachstum in Prozent** | | | | | | |
| **Leukämie** | | | | | | | |
| CCRF-CEM | 5,9 | 37,1 | - | - | - | 76,5 | - |
| HL-60(TB) | -11,6 | -16,1 | 3,1 | 76 | 70,3 | 78,8 | 102,7 |
| MOLT-4 | 0,5 | 24,6 | 14,2 | 89,4 | 75,3 | 85,9 | 86,2 |
| RPMI-8226 | 6,3 | 52,6 | - | - | 64,7 | 92,3 | - |
| SR | 0,4 | 10,6 | 3 | 91,8 | - | 41,4 | 75,5 |
| K-562 | - | - | 14,9 | 99,7 | 84,9 | - | 87,6 |
| **Mittelwert** | **0,3** | **21,8** | **8,8** | **89,2** | **73,8** | **75** | **70,4** |
| | | | | | | | |
| **Melanom** | | | | | | | |
| LOX IMVI | 38 | 44,6 | 44,2 | 89,7 | 95,7 | 86,3 | 107,3 |
| MALME-3M | - | 50 | 43,9 | 108,6 | 98,2 | 93,1 | 115,4 |
| M14 | 1,3 | 29,3 | 34,5 | 108,4 | 93,4 | 105,8 | 120,5 |
| MDA-MB-435 | -59,8 | 1,1 | -39 | 117,6 | 107 | 17,2 | 99,3 |
| SK-MEL-2 | 29,9 | 46,4 | -10,5 | 101,4 | 94,1 | 117,2 | 133,1 |
| SK-MEL-28 | 73,6 | 52,1 | 60,6 | 117 | 112,5 | 100,45 | 115,3 |
| SK-MEL-5 | 17,2 | 28 | -52,2 | 101,1 | 86,2 | 85,05 | 95,8 |
| UACC-257 | 48,7 | 88,5 | 66 | 106 | 107,8 | 110,9 | 110 |
| UACC-62 | 50,3 | 26,4 | 41,4 | 96,8 | 81,7 | 95,5 | 104,1 |
| **Mittelwert** | **24,9** | **40,7** | **14,3** | **105,2** | **97,4** | **90,2** | **100,1** |
| | | | | | | | |
| **Brustkrebs** | | | | | | | |
| MCF7 | 27,8 | 25,3 | 19,5 | 91,2 | 83,4 | 81,6 | 88,6 |
| MDA-MB-231/ATCC | 68,2 | 52,7 | 63 | 106 | 80,6 | 91,5 | 106,9 |
| HS-578T | 20,3 | 51,3 | -0,7 | - | - | 82,3 | 111,1 |
| BT-549 | 29,7 | 53,5 | 44,7 | 103,5 | 67,8 | 91,6 | 144,3 |
| T-47D | 75,5 | 60,3 | 57,4 | 98,9 | 84,6 | 104,9 | 111,8 |
| MDA-MB-468 | -4,5 | 21 | -22,3 | 103,3 | 87,6 | 48,9 | 104,5 |
| **Mittelwert** | **36,2** | **44** | **30,3** | **100,6** | **80,8** | **83,5** | **111,2** |
| | | | | | | | |
| **Mittelwert über alle 60 Zelllinien** | **34,1** | **33,4** | **46,1** | **102,1** | **90,5** | **109** | **106,5** |

Tabelle I: Zytotoxizitätstests des NCI an 60 Tumorzelllinien (c = 1 µM) mit Derivaten der Formeln I und II.

Die ersten drei Derivate zeigen deutliche Wachstumshemmungen auf die Zellen der für das Beispiel ausgewählten Tumorarten, während die restlichen 4 Derivate keine bis nur sehr geringe wachstumshemmende Effekte auf diese Zellen haben. Hieraus lässt sich ableiten, dass ein 3-Halogenphenylrest in 11-Position für die wachstumshemmende Wirkung der Phenanthrolinderivate der Formeln I und II essentiell ist. Weder Derivate mit Halogenatomen in 2- oder 4-Position (im Beispiel 2-Bromphenyl, bzw. 4-Bromphenyl), noch Derivate mit anderen funktionellen Gruppen in 3-Position des Phenylrings, noch der unsubstituierte Phenylring zeigen wachstumshemmende Effekte in diesem Ausmaß. *Tabelle II* zeigt die Ergebnisse dieses Tests, ausgewählt für 5 verschiedene Derivate der erfindungsgemäßen Phenanthrolinderivate der Formel III mit unterschiedlichen Resten in 11-Position. Die Reste in 11-Position sind in der

ersten Zeile dargestellt. Die linke Spalte zeigt die verwendeten Zelllinien aus 3 ausgewählten Tumorarten, Leukämie, Melanom, sowie Brustkrebs. Die Tabelle macht Aussagen über wachstumshemmende Wirkungen der entsprechenden Derivate mit ihren unterschiedlichen Resten.

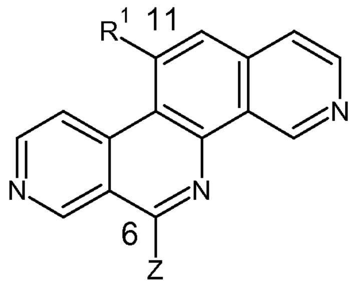

Strukturformel der am National Cancer Institute (NCI) getesteten Verbindungen der erfindungsgemäßen Phenanthrolinderivate der Formel III.

| Rest in 11-Position | 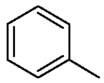 | 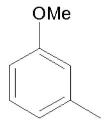 | 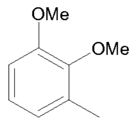 | 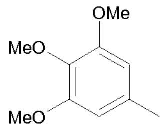 | 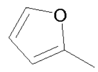 |
|---|---|---|---|---|---|
| **Zelllinien** | **Zellwachstum in Prozent** | | | | |
| **Leukämie** | | | | | |
| CCRF-CEM | 21,7 | 32,1 | 74,9 | 7,2 | 12,4 |
| HL-60(TB) | 18,9 | 26,0 | 118,7 | -0,9 | 13,9 |
| MOLT-4 | 2,3 | 11,9 | 90,1 | -8,9 | -0,9 |
| RPMI-8226 | 80,7 | 76,2 | 96,8 | 33,5 | 62,7 |
| SR | 11,1 | 15,3 | 83,5 | 13,1 | 7,8 |
| K-562 | 80,0 | 68,9 | 88,8 | 29,9 | 51,6 |
| **Mittelwert** | **35,8** | **38,4** | **92,1** | **12,3** | **24,6** |
| | | | | | |
| **Melanom** | | | | | |
| LOX IMVI | 41,0 | 37,9 | 89,3 | -30,4 | 2,3 |
| MALME-3M | 91,0 | 73,7 | 123,8 | 35,5 | 59,7 |
| M14 | 74,7 | 59,1 | 94,8 | 9,5 | 37,0 |
| MDA-MB-435 | - | - | - | - | - |
| SK-MEL-2 | 141,3 | 103,9 | 117,3 | 65,3 | 91,8 |
| SK-MEL-28 | 102,1 | 69,4 | 119,5 | 41,5 | 42,4 |
| SK-MEL-5 | 64 | 51,2 | 86,3 | 2,2 | 20,0 |
| UACC-257 | 124,5 | 78,0 | 126,3 | 30,9 | 69,8 |
| UACC-62 | 79,3 | 45,2 | 99,1 | - | 41,0 |
| **Mittelwert** | **89,9** | **64,8** | **95,2** | **22,1** | **45,5** |
| | | | | | |
| **Brustkrebs** | | | | | |
| MCF7 | 39,8 | 37,1 | 89,9 | 22,8 | 25,0 |
| MDA-MB-231/ATCC | 112,0 | 77,1 | 90,8 | - | 74,9 |
| HS-578T | 83,9 | 64,2 | 90,5 | -2,5 | - |
| BT-549 | 59,4 | 61,4 | 95,0 | 8,31 | 16,2 |
| T-47D | 97,0 | 77,7 | 97,4 | 36 | 48,4 |
| MDA-MB-468 | 98,2 | 53,7 | 81,5 | -23,6 | 35,6 |
| **Mittelwert** | **81,7** | **61,9** | **90,9** | **8,2** | **40,0** |
| | | | | | |
| **Mittelwert über alle 60 Zelllinien** | **69,9** | **55,6** | **98,0** | **16,8** | **39,1** |

Tabelle II: Zytotoxizitätstests des NCI an 60 Tumorzelllinien (c = 1 µM) mit Derivaten der Formel III.

Die Tabelle II zeigt die besten Ergebnisse hinsichtlich einer wachstumshemmenden Wirkung für das Derivat mit dem Trimethoxyphenyl-Rest in 11-Position. Das Derivat mit nur einer Methoxygruppe in meta-Position erzielt ebenfalls beachtliche Wachstumshemmungen. Das 11-Phenyl-Derivat hat nur eine geringe Wirkung, während sich eine Substitution in o-Position am Beispiel des 2,3-Dimethoxyphenyl-Derivates als nachteilig erweist. Hieraus lässt sich ableiten, dass zumindest in der meta-Position des 11-Phenylrings ein Substituent vorhanden sein muss. Durch die Ergebnisse der Tabelle I ist zu erwarten, dass der Ersatz von Methoxy- durch Halogen in Meta-Position ebenfalls zu guten wachstumshemmenden Effekten führt. Stellvertretend für einen fünfgliedrigen Heteroaromaten zeigt hier das 11-Furyl-Derivat

gute zytotoxische Effekte.

Des Weiteren ist aus den Tabellen I und II zu entnehmen, dass die hier getesteten erfindungsgemäßen Phenanthrolinderivate Selektivitäten für bestimmte Tumorarten besitzen. Am deutlichsten ausgeprägt ist eine wachstumshemmende Wirkung auf Tumorzellen der Leukämie. Ferner ist eine gewisse Selektivität für Tumorzellen des Melanoms und des Mammakarzinoms (Brustkrebs) zu erkennen. Diese Ergebnisse sind repräsentativ für alle 60 Zelllinien. Von den wirksamsten Verbindungen sind des Weiteren Dosis-Wirkungs-Beziehungen für 5 Konzentrationen bestimmt worden. Die $GI_{50}$ (growth inhibition 50%) beschreibt dabei die Konzentration der Testsubstanz, die für eine Hemmung des Zellwachstums um 50% erforderlich ist. Daraus wird der sog. "meangraph-midpoint" (MG_MID) ermittelt, der als Mittelwert der logarithmierten $GI_{50}$-Werte einem durchschnittlichen Ansprechverhalten aller 60 Zelllinien auf die untersuchte Testsubstanz entspricht [Boyd und Paull, Drug. Develop. Res., 34, (1995) 91-109,]

$$MG_{MID} = \frac{\sum \log GI_{50}}{60}$$

Mit dieser Größe lässt sich die Aktivität einer Verbindung in dem verwendeten Testsystem charakterisieren und quantitativ mit der Aktivität anderer Verbindungen vergleichen (Tabelle 3). Die folgende Tabelle zeigt 4 Derivate der Formeln I und III mit ihren verwendeten Codes, die für die entsprechenden Substanzen ermittelten meangraph-midpoints und die daraus errechneten $GI_{50}$-Werte.

Legende zur Tabelle 3:

| Code | MG_MID | $\sum \frac{GI_{50}}{60}$ [μM] |
|---|---|---|
| P4 | -5,09 | 8,13 |
| P5 | -5,57 | 2,69 |
| P5-O | -5,07 | 8,51 |
| P16 | -5,42 | 3,80 |
| P26 | -5,22 | 6,02 |
| Fagaronin | -5,00 | 9,48 |

P4 = 6-Amino-11,12-dihydropyrido[3,4-c][1,9]phenanthrolin, P5 = 6-Amino-11-(3-bromphenyl)-11,12-dihydropyrido-[3,4-c][1,9]phenanthrolin, P5-O = 11-(3-bromphenyl)-6-oxo-5,6,11,12-tetrahydropyrido[3,4-c][1,9]phenanthrolin, P16 = 6-Amino-11-(3-chlorphenyl)-11,12-dihydropyrido-[3,4-c][1,9]phenanthrolin, P26 = 6-Amino-11-(3,4-di-chlorphenyl)-11,12-dihydropyrido-[3,4-c]-[1,9]phenanthrolin.

Zusätzlich zu den erfindungsmäßen Verbindungen wurde Fagaronin getestet, ein pflanzliches Benzo[*c*]phenanthridin-Alkaloid, das als Leitsubstanz für die Entwicklung synthetischer Benzo[c]phenanthridin-Derivate gilt (Pommier, Y. Nat. Rev. Cancer, 6, (2006), 789; Pommier, Y. Chem. Rev. 109, (2009), 2984). Festzuhalten ist, dass alle Derivate der erfindungsgemäßen Phenathroline, für die Dosis-Wirkungskurven bestimmt worden sind, eine bessere wachstumshemmende Wirkung aufweisen, als der Naturstoff Fagaronin.

Beispielhaft für 6-Amino-11-(3-bromphenyl)-11,12-dihydropyrido[3,4-*c*][1,9]phenanthrolin sind die Dosis-Wirkungs-Kurven in der in den Abbildungen 1 bis 9 wiedergegeben. Die neun verschiedenen Abbildungen beinhalten die verschiedenen Krebsformen. Aufgetragen ist jeweils das prozentuale Wachstum in Abhängigkeit von der Konzentration der Verbindung (als $\log_{10}$ der molaren Konzentrationen). Die einzelnen Kurven einer jeden Krebsart sind verschiedene Zelllinien dieser Krebsform, die als Legenden in ihren üblichen Abkürzungen erscheinen. Horizontale Linien in den Abbildungen bedeuten Prozentwachstum +100, +50, 0, -50, -100. 100 % Wachstum bedeutet z.B. keinen Unterschied zum Wachstum nach zwei Tagen ohne Substanzzusatz. Man erkennt bei den einzelnen Kurven, dass sich mit steigenden Konzentrationen der Substanz das Prozentwachstum verkleinert.

Ein besonderer Vorteil der erfindungsgemäßen Phenanthrolinderivate ist ihre verbesserte Wasserlöslichkeit im Vergleich zu den 6-Aminobenzo[c]phenanthridinen. Hierbei ist vor allem die Löslichkeit in sauren wässrigen Medien zu nennen (2-20 mM in Phosphatpuffer pH=2, und noch 0,01-0,5 mmol in Phosphatpuffer pH=4), welche auf 2 zusätzliche, protonierbare Stickstoffatome zurückzuführen ist. Bei pH 7,4 konnten für zahlreiche Derivate Löslichkeiten im unteren mikromolaren Bereich (<1-15 μM) gefunden werden, die Löslichkeit der 6-Aminobenzo[*c*]phenanthridine bei diesem pH-Wert liegt vorherrschend im nanomolaren Bereich. Die Abbildung 10 zeigt 4 Einzelgrafiken, stehend für 4 Phosphatpuffersysteme mit verschiedenen pH-Werten, in denen jeweils die Löslichkeiten der erfindungsgemäßen 6-Amino-11,12-dihydropyrido[3,4-c][1,9]phenanthroline auf der y-Achse aufgetragen sind, während auf der x-Achse die verschiedene Derivate mit unterschiedlichen Resten in 11-Position (3a: R = phenyl, 3b: R = 3-methoxyphenyl, 3d: R = 2,3-dimethoxyphenyl, 3e: R = 3,5-dimethoxyphenyl, 3f: R = 3,4,5-trimethoxyphenyl, 3g: R = 2,4,6-trimethoxyphenyl, 3h: R = furyl, 3i: R = thienyl, 3j: R = 3-bromophenyl, 3k: R = 4-bromophenyl, 31: R = 3-chlorophenyl, 3m: R = 4-fluorophenyl, 3n: R = biphenyl, 3o: R = propyl) zu sehen sind.

Von den erfindungsgemäßen Phenanthrolinderivaten wurden des Weiteren logP-Werte als ein Maß für die Lipophilie der Substanzklasse bestimmt. Hierbei wurden sechs unterschiedliche 6-Amino-11,12-dihydropyrido[3,4-c][1,9]phenanthroline mit unterschiedlichen Resten in 11-Position mit den korrespondierenden Benzo[c]phenanthridinen verglichen. Die Abbildung 11 zeigt die logP-Werte auf der y-Achse, aufgetragen gegen die Derivate, die sich in den sechs benannten Resten in 11-Position unterscheiden, jeweils für die beiden verglichenen Substanzklassen. Desweiteren ist der Mittelwert (MW) aufgetragen.

Aus der Grafik wird ersichtlich, dass die logP-Werte der erfindungsgemäßen 6-Amino-11,12-dihydropyrido[3,4-*c*][1,9]phenanthroline im Mittel ungefähr um den Wert 2 niedriger sind als bei den entsprechenden Benzo[c]phenanthridinen.

Dies ist von enormer Wichtigkeit, betrachtet man die Eignung der Substanzklasse als Arzneistoff und vor allem seine orale Bioverfügbarkeit. Nach der "Rule of Five" von *Lipinski* (Lipinski, C. A.; Lombardo, F.; Dominy, B. W.; Feeney, P. J. Adv. Drug. Deliv. Rev. 46 (2001), 3-26), einer allgemein anerkannten Faustregel für die Abschätzung der oralen Bioverfügbarkeit eines möglichen Arzneistoffs, ist ein Stoff oral bioverfügbar, wenn er nicht mehr als fünf Donatoren von Wasserstoffbrückenbindungen (z. B. OH- oder NH-Gruppen), nicht mehr als zehn Akzeptoren von Wasserstoffbrückenbindungen (z. B. Sauerstoff- oder Stickstoffatome), eine Molekülmasse von nicht mehr als 500 g/mol, sowie einen Verteilungskoeffizienten (logP) zwischen Oktanol und Wasser (Oktanol-Wasser-Verteilungs-koeffizient) von maximal 5 besitzt. Die untersuchten erfindungsgemäßen Derivate liegen alle innerhalb der Grenzbereiche der "Rule of Five" und eignen sich danach als oral applizierbare Arzneistoffe, z.B. in Form von Lösungen, Tabletten, Kapseln, etc.

Die Abbildungen zeigen Dosis-Wirkungs-Kurven von 9 verschiedenen Tumorarten aus dem 5-dose-assay (NCI) für 6-Amino-11-(3-bromphenyl)-11,12-dihydropyrido[3,4-*c*][1,9]phenanthrolin.

**Kurze Beschreibung der Abbildungen**

Abb. 1: Dosis-Wirkungs-Kurven für Leukämie-Tumore aus dem 5-dose-assay (NCI) für 6-Amino-11-(3-bromphenyl)-11,12-dihydropyrido[3,4-*c*][1,9]phenanthrolin

Abb. 2: Dosis-Wirkungs-Kurven für nicht-kleinzellige Bronchialkarzinome aus dem 5-dose-assay (NCI) für 6-Amino-11-(3-bromphenyl)-11,12-dihydropyrido[3,4-c] [1,9]phenanthrolin

Abb. 3: Dosis-Wirkungs-Kurven für Colonkarzinome aus dem 5-dose-assay (NCI) für 6-Amino-11-(3-bromphenyl)-11,12-dihydropyrido[3,4-*c*][1,9]phenanthrolin

Abb. 4: Dosis-Wirkungs-Kurven für ZNS-Krebs aus dem 5-dose-assay (NCI) für 6-Amino-11-(3-bromphenyl)-11,12-dihydropyrido[3,4-*c*][1,9]phenanthrolin

Abb. 5: Dosis-Wirkungs-Kurven für Melanome aus dem 5-dose-assay (NCI) für 6-Amino-11-(3-bromphenyl)-11,12-dihydropyrido[3,4-*c*][1,9]phenanthrolin

Abb. 6: Dosis-Wirkungs-Kurven für Nierenzellkarzinome aus dem 5-dose-assay (NCI) für 6-Amino-11-(3-bromphenyl)-11,12-dihydropyrido[3,4-*c*][1,9]phenanthrolin

Abb. 7: Dosis-Wirkungs-Kurven für Ovarialkarzinome aus dem 5-dose-assay (NCI) für 6-Amino-11-(3-bromphenyl)-11,12-dihydropyrido[3,4-*c*][1,9]phenanthrolin

Abb. 8: Dosis-Wirkungs-Kurven für Prostatakarzinome aus dem 5-dose-assay (NCI) für 6-Amino-11-(3-bromphenyl)-11,12-dihydropyrido[3,4-*c*][1,9]phenanthrolin

Abb. 9: Dosis-Wirkungs-Kurven für Mammakarzinome aus dem 5-dose-assay (NCI) für 6-Amino-11-(3-bromphenyl)-11,12-dihydropyrido[3,4-*c*][1,9]phenanthrolin

Abb. 10: pH-abhängige Löslichkeiten der 6-Amino-11,12-dihydropyrido[3,4-c] [1,9]phenanthroline

Abb. 11: logP-Werte in Abhängigkeit von den Resten in 11-Position

**Patentansprüche**

**1.** Verbindung der allgemeinen Formel I oder II

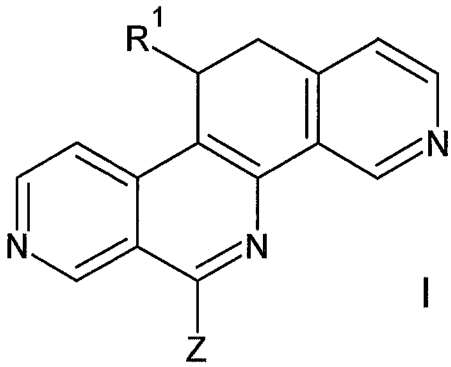
I

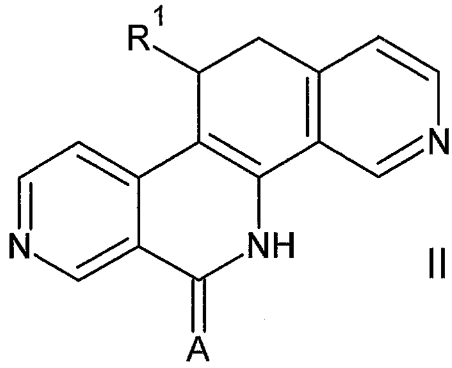
II

wobei $R^1$ gleich einem Phenylring der Formel

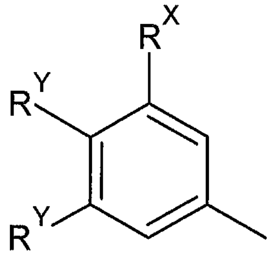

ist, der mindestens einen Substituenten $R^X$ ausgewählt aus der Gruppe Cl, Br oder I in meta-Stellung trägt, wobei $R^Y$, welches gleich oder verschieden sein kann, gleich H, OH, OMe, OEt oder Halogen ist; sowie Verbindung der allgemeinen Formel III oder IV,

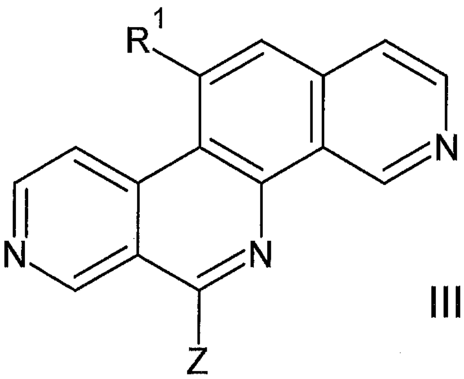
III

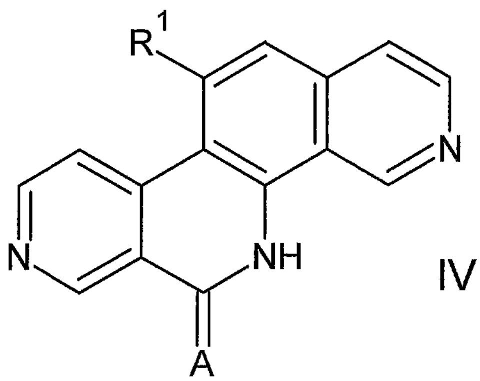
IV

wobei $R^1$ entweder gleich einem Phenylring der Formel

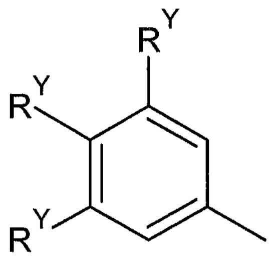

ist, der mindestens einen Substituenten $R^Y$ in meta-Stellung trägt, wobei $R^Y$, welches gleich oder verschieden sein kann, gleich H, OH, OMe, OEt oder Halogen ist oder gleich einem fünfgliedrigen aromatischen Heterocyclus der Formel

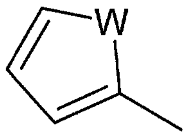

ist,

wobei W gleich O, S oder NH ist; und
wobei Z gleich H, F, Cl, Br, I, NHX, OX, SX
wobei X gleich einem H, Dimethylaminoalkyl-, Diethyaminoalkyl-, ω-(1,3-diazol-1-yl)-alkyl-, Hydroxyalkyl-, Alkoxyalkyl-, Thiolalkyl-, Alkylthioalkylgruppe ist,
wobei Alkyl gleich Methyl, Ethyl oder Propyl
und wobei A gleich O oder S ist,
sowie deren pharmazeutisch verträgliche Salze und Solvate.

**2.** Verfahren zur Herstellung einer Verbindung nach Anspruch 1, umfassend mindestens die folgenden Schritte:

(i) Umsetzung von substituierten Aldehyden der allgemeinen Formel $R^1$ -CHO (V) mit substituierten 4-Methylpyridin-3-carbonitrilen der allgemeinen Formel

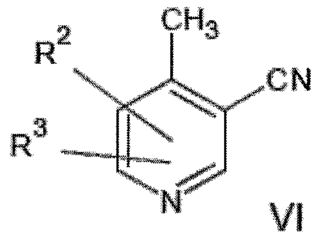

VI

in aprotischen bipolaren Lösungsmitteln in Gegenwart von Basen zu einer Verbindung der allgemeinen Formel

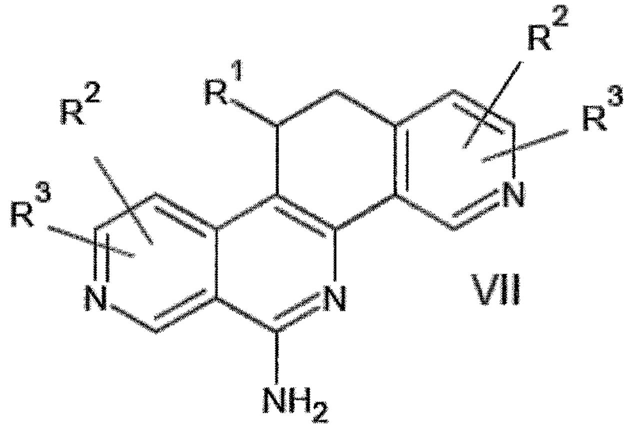

VII

(ii) Isolierung der Produkte und Derivatisierung an Position 6 unter Einfügen der Reste $R^4$ zu den Derivaten mit A- oder Z-Substitution in 6-Position, gemäß der Formel I bis IV, wobei

$R^1$ ein H, ein cyclischer oder acyclischer, verzweigter oder unverzweigter aliphatischer Kohlenwasserstoffrest, einfach oder mehrfach substituiert, ein aromatischer carbocyclischer oder heterocyclischer Rest, einfach oder mehrfach substituiert ist, und
$R^2$ und $R^3$, gleich oder verschieden sein können und ein H, Alkyloxyrest, Alkylenoxyrest, Halogenatom oder eine Nitrogruppe ist, und
$R^4$ ein H, eine Monoamino-, Alkylamino-, Dialkylamino-, (Dialkyl-)aminoalkylamin-, Alkyl-, Alkoxy-, (Dialkyl-)aminoalkyloxy-, Hydroxy-, Hydroxyalkylamino-, Hydroxyalkyloxy-, Thiol-, (Dialkyl-)aminoalkylthio-, Thiolalkyl-, Alkylthioalkylgruppe oder ein Halogenatom ist.

**3.** Verfahren nach Anspruch 2, wobei zwischen den beiden Schritten (i) und (ii) optional der Verfahrensschritt

(i.a.) Dehydrierung und Isolierung der hieraus resultierenden Produkte der allgemeinen Formel

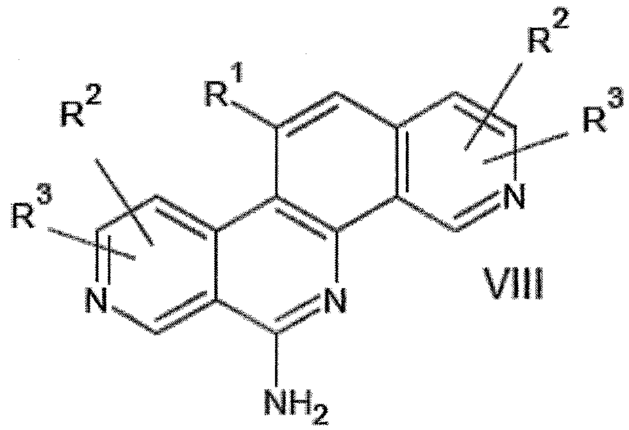

VIII

eingeführt sein kann.

**4.** Verfahren nach Anspruch 2 oder 3, wobei das aprotische dipolare Lösungsmittel vorzugsweise ein Amid wie Dimethylformamid, Dimethylacetamid, Diethylacetamid, Hexamethylphosphorsäuretrisamid oder ein Harnstoff wie Tetramethylharnstoff, 1,3-Dimethyltetrahydropyrimidin-2-on und 1,3-Dimethylimidazolidinon oder ein Dimethylsulfoxid ist, und
wobei die Base ein Alkali- oder Erdalkalihydrid wie Natriumhydrid, Alkaliamid wie Natriumamid, Natriummethylacetamid, Alkali-, Erdalkali- oder Aluminiumalkoholat wie Kalium-tert-butylat, Natriummethylat, Natriumethylat oder Aluminiumethylat ist.

**5.** Verbindung nach Anspruch 1 zur Verwendung als Arzneimittel.

**6.** Verbindung nach Anspruch 1 zur Verwendung bei der Behandlung einer Erkrankung ausgewählt aus einer Gruppe bestehend aus mikrobiellen, fungiziden, viralen und/oder inflammatorischen Erkrankungen.

**7.** Verbindung nach Anspruch 1 zur Verwendung bei der Behandlung von Krebs.

**8.** Verbindung nach Anspruch 7, wobei der Krebs ausgewählt ist aus einer Gruppe bestehend aus Leukämie, Melanom oder Mammakarzinom.

**9.** Pharmazeutische Zusammensetzung enthaltend mindestens eine Verbindung nach einem der Ansprüche 1 oder 5 bis 8 in Kombination mit einem geeigneten Hilfsstoff.

**Claims**

**1.** A compound of general formula I or II

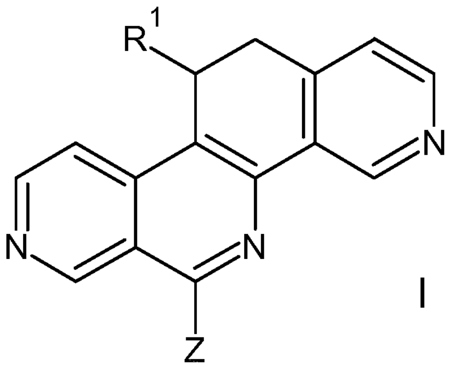
I

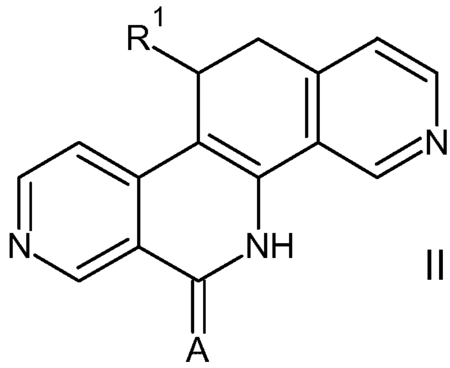
II

wherein $R^1$ is equal to a phenyl ring of the formula

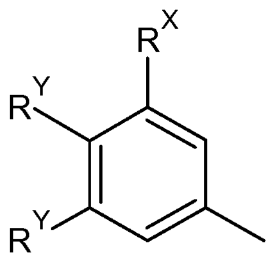

which carries at least one substituent $R^X$ selected from the group Cl, Br or I in meta position, wherein $R^Y$, which may be identical or different, is equal to H, OH, OMe, OEt or halogen; as well as compound of the general formula III or IV,

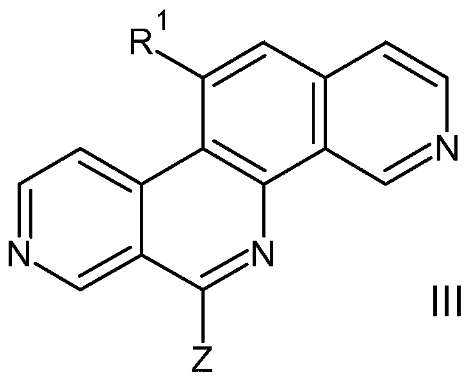
III

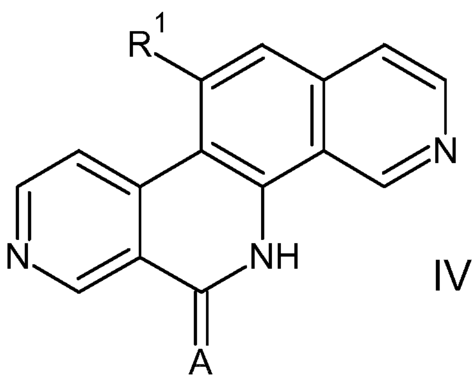
IV

wherein $R^1$ is either equal to a phenyl ring of the formula

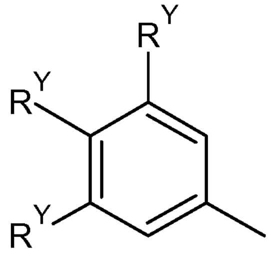

which carries at least one substituent $R^Y$ in meta position, wherein $R^Y$, which may be identical or different, is equal to H, OH, OMe, OEt or halogen or is equal to a five-membered aromatic heterocycle of the formula

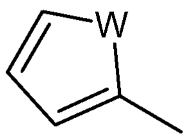

wherein W is equal to O, S or NH; and
wherein Z is equal to H, F, Cl, Br, I, NHX, OX, SX
wherein X is equal to H, dimethylaminoalkyl, diethylaminoalkyl, ω-(1,3-diazol-1-yl)-alkyl, hydroxyalkyl, alkoxyalkyl, thioalkyl, alkylthioalkylgroup,
wherein alkyl is equal to methyl, ethyl or propyl
and wherein A is equal to O or S,
as well as their pharmaceutically acceptable salts and solvates.

**2.** A process for preparing a compound according to claim 1, comprising at least the following steps:

(i) conversion of substituted aldehydes of general formula $R^1$ - CHO (V) with substituted 4-methylpyridine-3-carbonitriles of the general formula

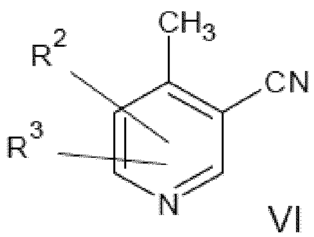

VI

in aprotic bipolar solvents in the presence of bases to a compound of the general formula

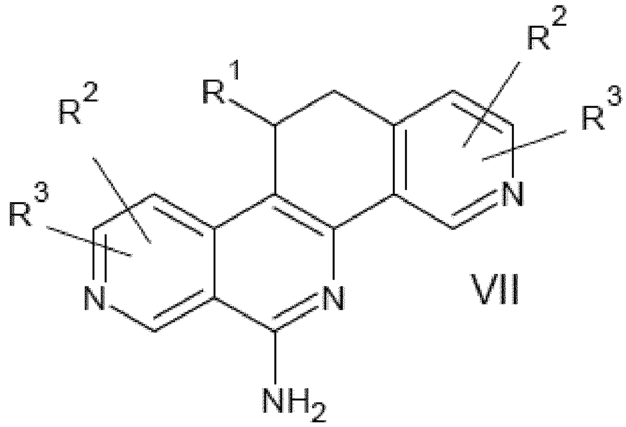

VII

(ii) isolation of the products and derivatization at position 6 with insertion of the residues $R^4$ to the derivatives with A or Z substitution at position 6, according to formula I to IV, wherein

$R^1$ is a H, a cyclic or acyclic, branched or unbranched aliphatic hydrocarbon residue, singly or multiply substituted, an aromatic carbocyclic or heterocyclic residue, singly or multiply substituted, and
$R^2$ and $R^3$, may be identical or different and is H, alkyloxy residue, alkylenoxy residue, a halogen atom or a nitro group, and
$R^4$ is a H, a monoamino, alkylamino, dialkylamino, (dialkyl)aminoalkylamine, alkyl, alkoxy, (dialkyl)aminoalkyloxy, hydroxy, hydroxyalkylamino, hydroxyalkyloxy, thiol, (dialkyl)aminoalkylthio, thioalkyl, alkylthioalkyl group or a halogen atom.

**3.** A method according to claim 2, wherein between the two steps (i) and (ii) optionally the method step

(i.a.) dehydration and isolation of the thereof resulting products of the general formula

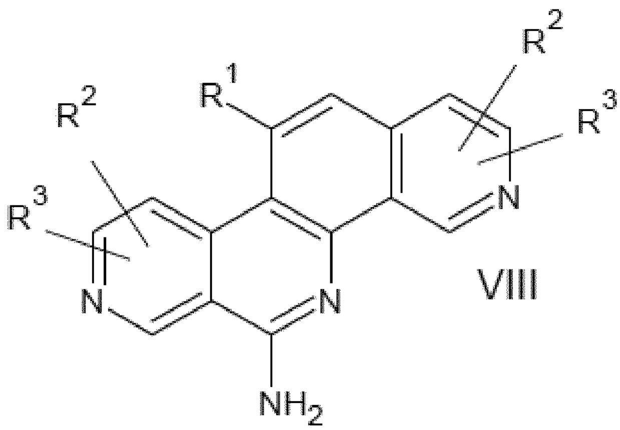

VIII

may be introduced.

**4.** The method according to claim 2 or 3, wherein the aprotic dipolar solvent is preferably an amide such as dimethylformamide, dimethylacetamide, diethylacetamide, hexamethylphosphoracidtrisamide or a urea such as tetramethylurea, 1,3-dimethyltetrahydropyrimidin-2-one and 1,3-dimethylimidazolidinone or a dimethylsulfoxide, and wherein the base is an alkali or alkaline earth hydride such as sodium hydride, alkali amide such as sodium amide, sodiummethylacetamide, alkali, earth alkali or aluminum alcoholate such as potassium tert-butylate, sodium methylate, sodium ethylate or aluminum ethylate.

**5.** The compound according to claim 1 for use as a medicament.

**6.** The compound according to claim 1 for use in the treatment of a disease selected from the group consisting of microbial, fungicidal, viral and/or inflammatory diseases.

7. The compound according to claim 1 for use in the treatment of cancer.

8. A compound according to claim 7, wherein the cancer is selected from the group consisting of leukemia, melanoma or mamma carcinoma.

9. A pharmaceutical composition containing at least one compound according to any one of claims 1 or 5 to 8 in combination with a suitable excipient.

## Revendications

1. Composé de formule générale I ou II

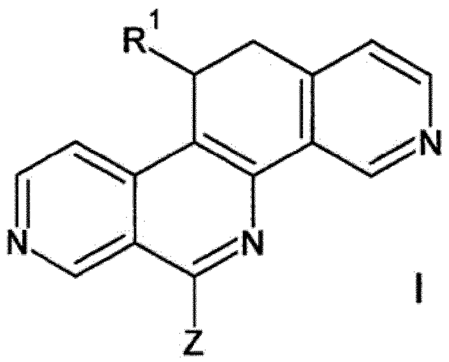

I

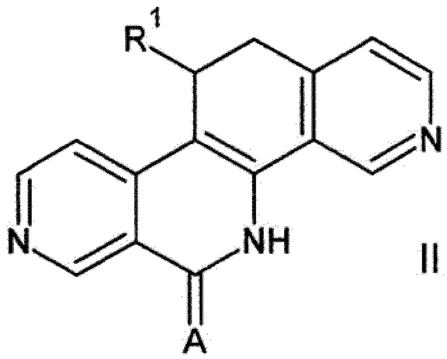

II

où $R^1$ est un cycle phényle de formule

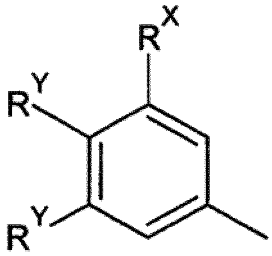

qui porte au moins un substituant $R^X$ choisi dans le groupe Cl, Br ou I en position méta, où $R^Y$, qui peut être identique ou différent, est H, OH, OMe, OEt ou halogène; ainsi que composé de formule générale III ou IV

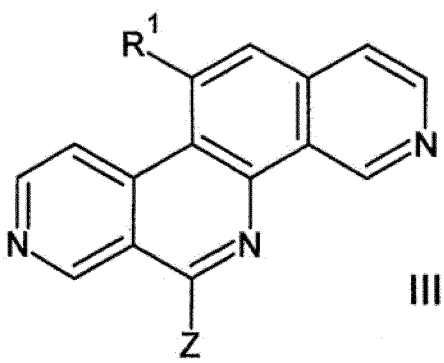

III

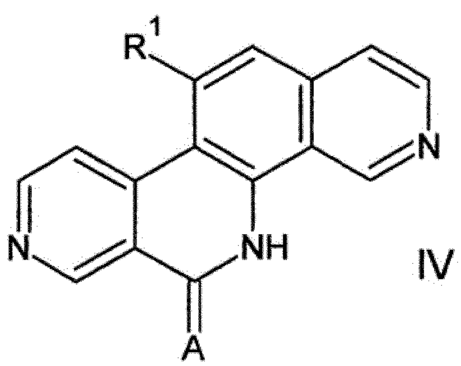

IV

où $R^1$ est un cycle phényle de formule

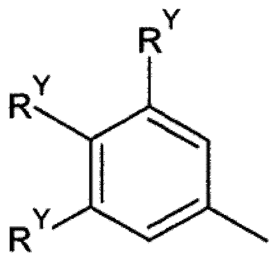

qui porte au moins un substituant $R^Y$ en position méta, où $R^Y$, qui peut être identique ou différent, est H, OH, OMe, OEt ou halogène ou un hétérocycle aromatique à cinq chaînons de formule

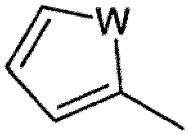

où W est O, S ou NH; et
où Z est H, F, Cl, Br, I, NHX, OX, SX
où X est un groupe H, diméthylaminoalkyle, diéthylaminoalkyle, ω-(1,3-diazol-1-yl)-alkyle, hydroxyalkyle, alcoxyalkyle, thiolalkyle, alkylthioalkyle,
où alkyle est méthyle, éthyle ou propyle
et où A est O ou S,
ainsi que leurs sels et solvates pharmaceutiquement acceptables.

**2.** Procédé de production d'un composé selon la revendication 1, comprenant au moins les étapes suivantes:

(i) réaction d'aldéhydes substitués de formule générale $R^1$-CHO (V) avec des 4-méthylpyridine-3-carbonitriles substitués de formule générale

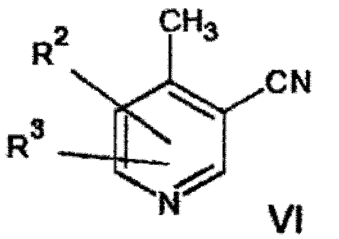

VI

dans des solvants bipolaires aprotiques en présence de bases en un composé de formule générale

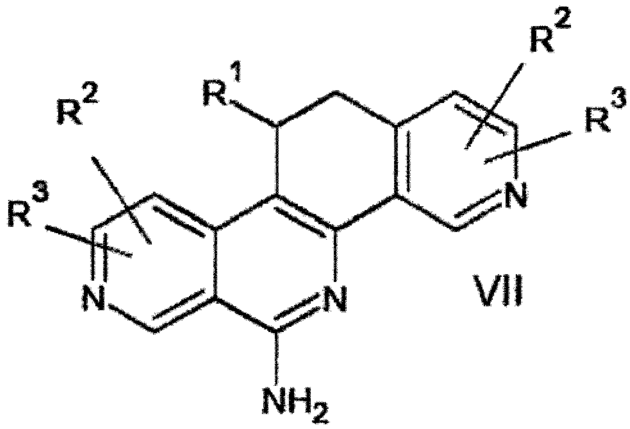

VII

(ii) isolement des produits et dérivatisation à la position 6 avec insertion des groupements $R^4$ en les dérivés avec une substitution A ou Z en position 6, selon les formules I à IV, où

$R^1$ est un H, un groupement hydrocarboné aliphatique ramifié ou non ramifié, cyclique ou acyclique, substitué une fois ou plusieurs fois, un groupement carbocyclique ou hétérocyclique aromatique, substitué une fois ou plusieurs fois, et
$R^2$ et $R^3$, peuvent être identiques ou différents et sont un H, un groupement alkyloxy, un groupement alkylèneoxy, un atome d'halogène ou un groupe nitro, et
$R^4$ est un H, un groupe monoamino, alkylamino, dialkylamino, (dialkyl)amino-alkylamino, alkyle, alcoxy, (dialkyl)aminoalkyloxy, hydroxy, hydroxyalkylamino, hydroxyalkyloxy, thiol, (dialkyl)aminoalkylthio, thiolalkyle, alkylthioalkyle ou un atome d'halogène.

3. Procédé selon la revendication 2, où entre les deux étapes (i) et (ii), l'étape de procédé

(i.a.) déshydratation et isolement des produits qui en résultent de formule générale

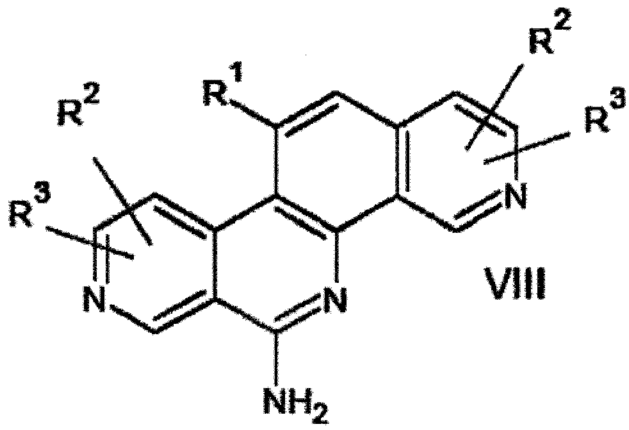

VIII

peut éventuellement être insérée.

4. Procédé selon la revendication 2 ou 3, où le solvant dipolaire aprotique est de préférence un amide comme le diméthylformamide, le diméthylacétamide, le diéthylacétamide, le trisamide hexaméthylphosphorique ou une urée comme la tétraméthylurée, la 1,3-diméthyltétrahydropyrimidin-2-one et la 1,3-diméthylimidazolidinone ou un diméthylsulfoxyde, et
où la base est un hydrure alcalin ou alcalino-terreux comme l'hydrure de sodium, un amidure alcalin comme l'amidure de sodium, le méthylacétamidure de sodium, un alcoolate alcalin, alcalino-terreux ou d'aluminium comme le tert-butylate de potassium, le méthylate de sodium, l'éthylate de sodium ou l'éthylate d'aluminium.

5. Composé selon la revendication 1 destiné à être utilisé comme médicament.

6. Composé selon la revendication 1 destiné à être utilisé dans le traitement d'une maladie choisie dans un groupe consistant en les maladies microbiennes, fongiques, virales et/ou inflammatoires.

7. Composé selon la revendication 1 destiné à être utilisé dans le traitement du cancer.

8. Composé selon la revendication 7, où le cancer est choisi dans un groupe consistant en la leucémie, le mélanome ou le carcinome mammaire.

9. Composition pharmaceutique contenant au moins un composé selon l'une des revendications 1 ou 5 à 8 en combinaison avec un auxiliaire approprié.

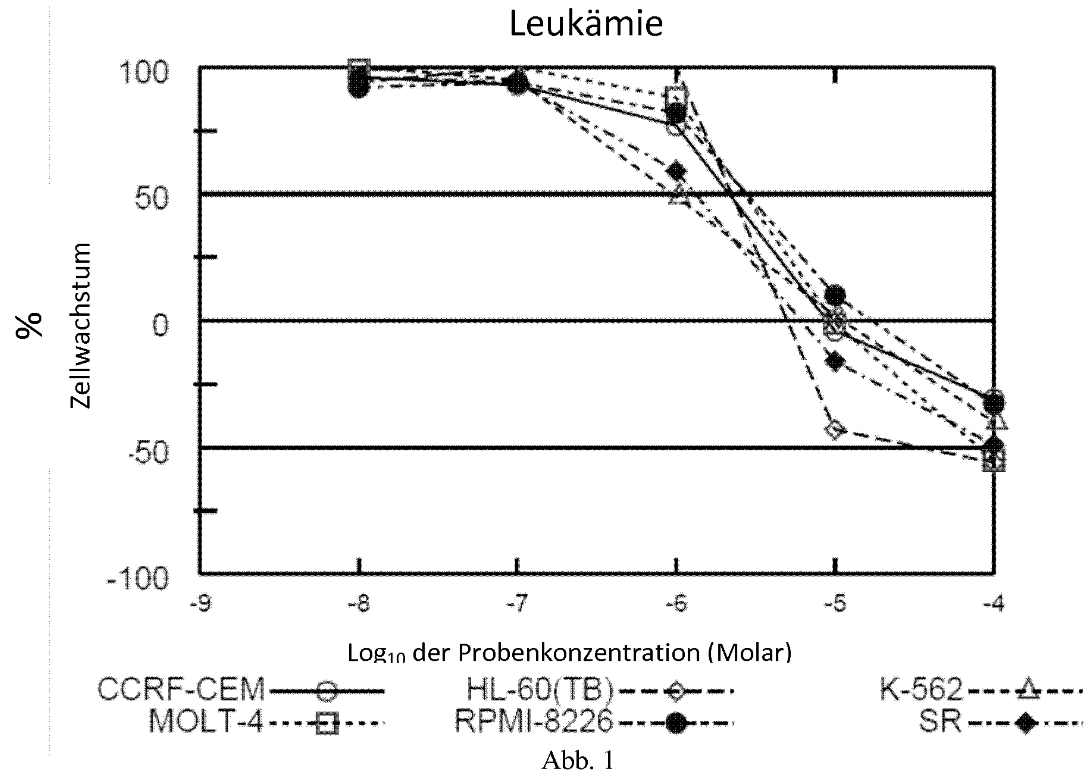
Leukämie
100
50
0
-50
-100
% Zellwachstum
-9
-8
-7
-6
-5
-4
Log10 der Probenkonzentration (Molar)
CCRF-CEM
HL-60(TB)
K-562
MOLT-4
RPMI-8226
SR

Abb. 1

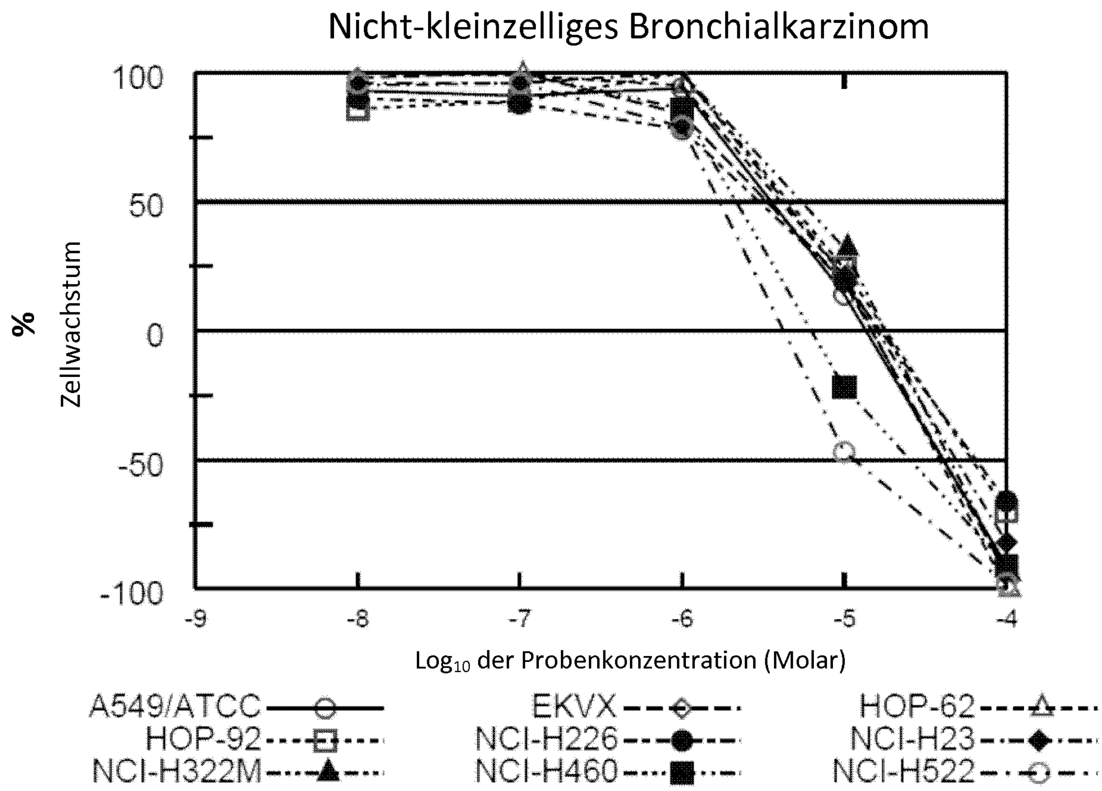
Nicht-kleinzelliges Bronchialkarzinom
100
50
0
-50
-100
% Zellwachstum
-9
-8
-7
-6
-5
-4
Log10 der Probenkonzentration (Molar)
A549/ATCC
EKVX
HOP-62
HOP-92
NCI-H226
NCI-H23
NCI-H322M
NCI-H460
NCI-H522

Abb. 2

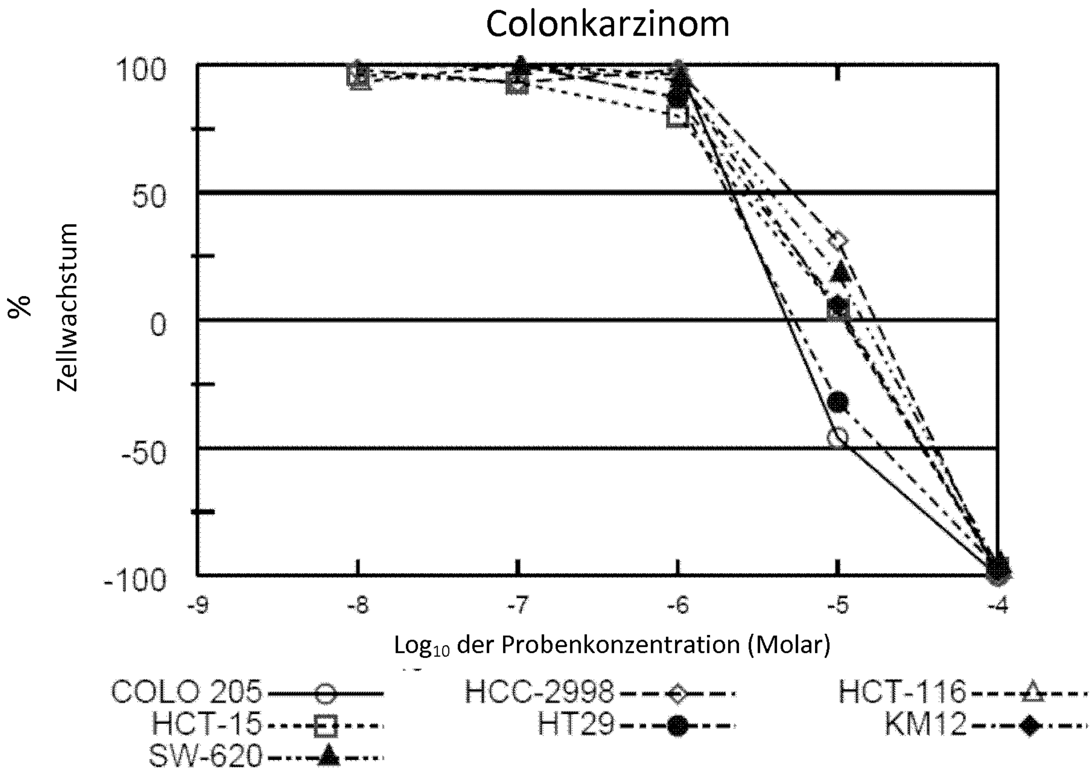
Colonkarzinom
100
50
0
-50
-100
% Zellwachstum
-9
-8
-7
-6
-5
-4
Log10 der Probenkonzentration (Molar)
COLO 205
HCC-2998
HCT-116
HCT-15
HT29
KM12
SW-620

Abb. 3

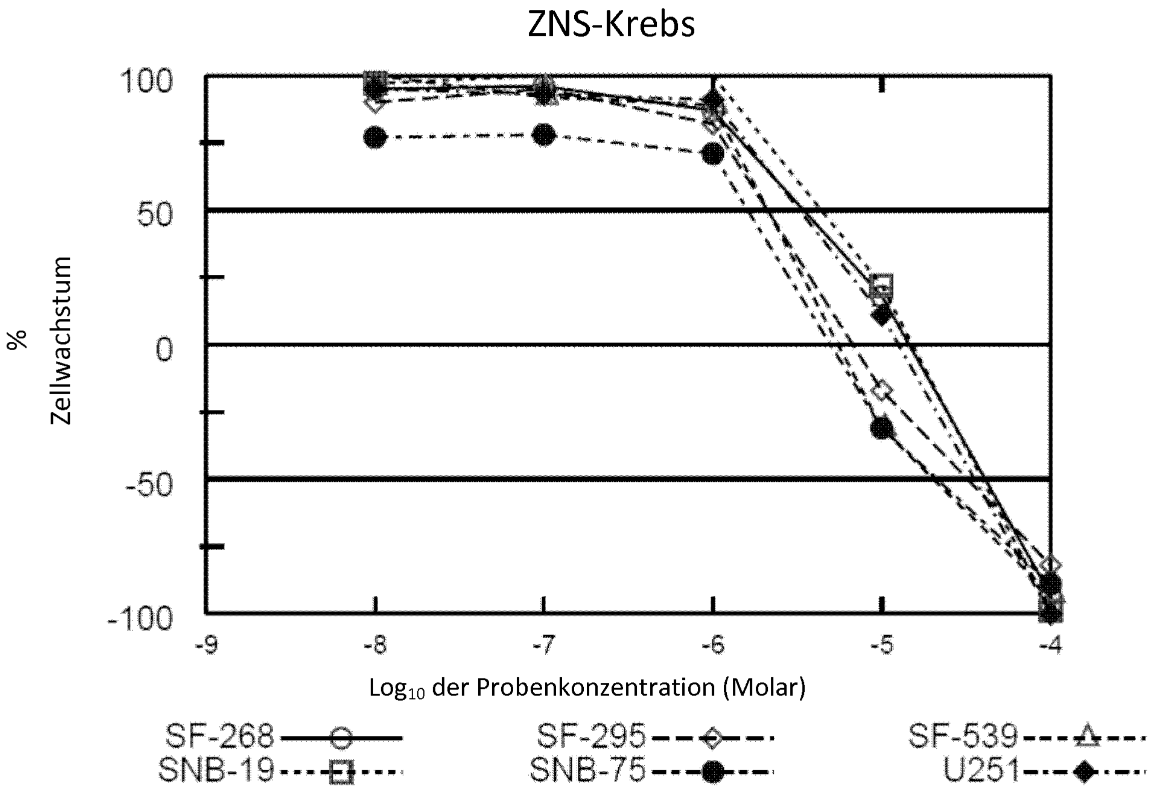
ZNS-Krebs
100
50
0
-50
-100
% Zellwachstum
-9
-8
-7
-6
-5
-4
Log10 der Probenkonzentration (Molar)
SF-268
SF-295
SF-539
SNB-19
SNB-75
U251

Abb. 4

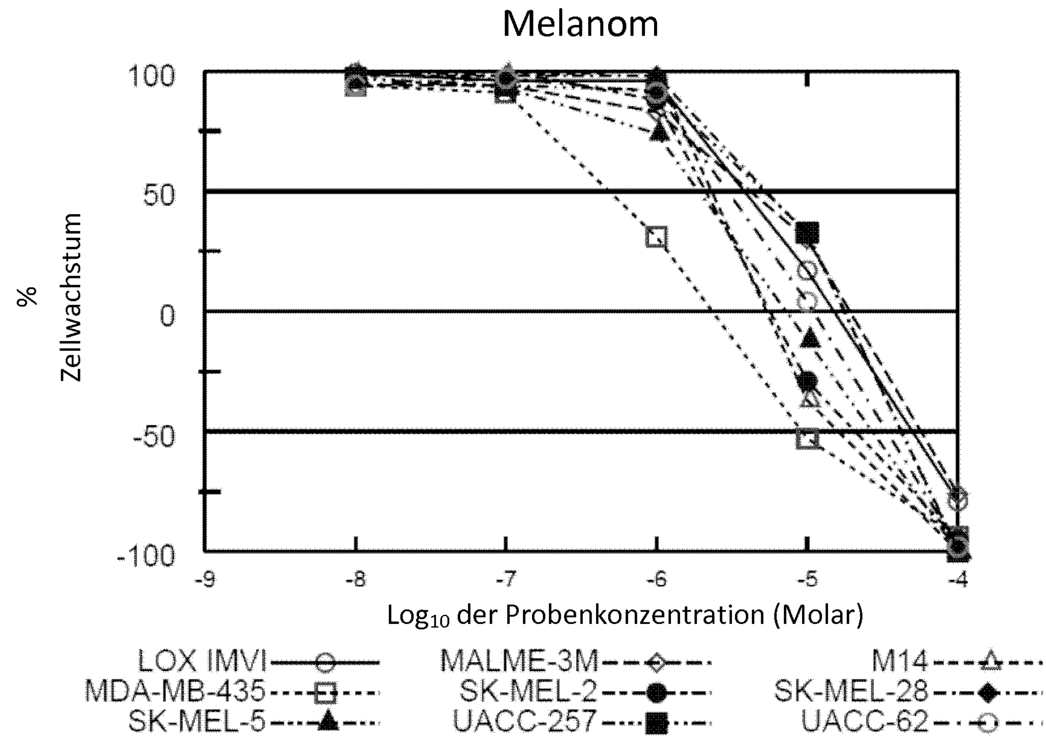
Melanom
100
50
0
-50
-100
% Zellwachstum
-9
-8
-7
-6
-5
-4
Log10 der Probenkonzentration (Molar)
LOX IMVI
MALME-3M
M14
MDA-MB-435
SK-MEL-2
SK-MEL-28
SK-MEL-5
UACC-257
UACC-62

Abb. 5

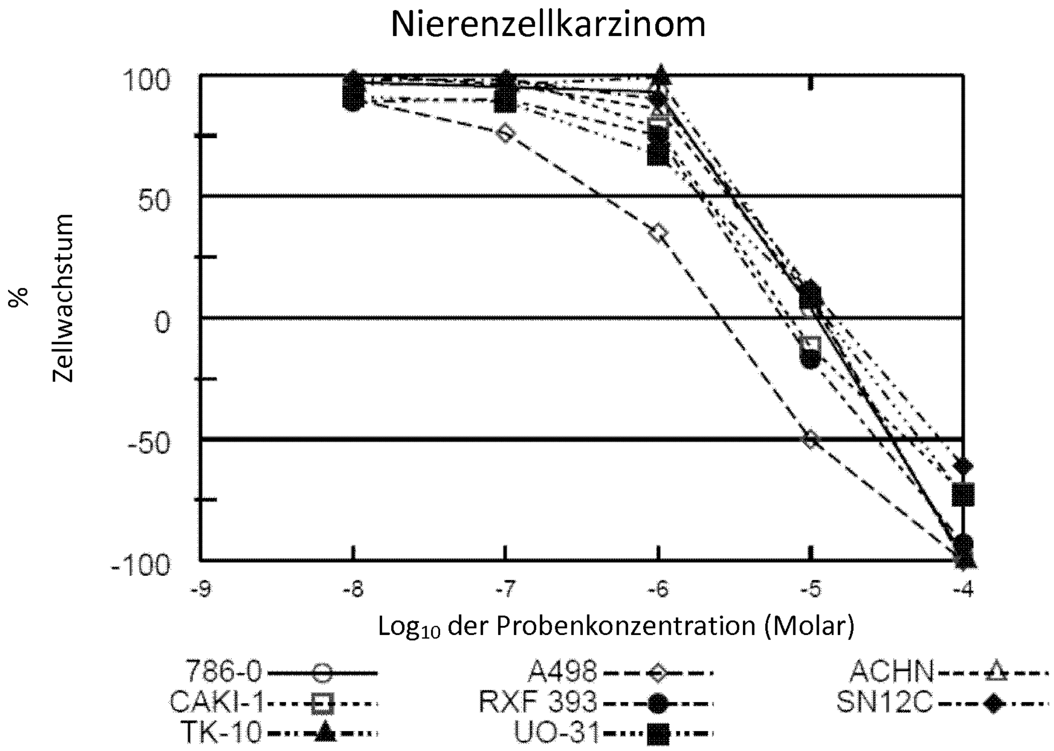
Nierenzellkarzinom
100
50
0
-50
-100
% Zellwachstum
-9
-8
-7
-6
-5
-4
Log10 der Probenkonzentration (Molar)
786-0
A498
ACHN
CAKI-1
RXF 393
SN12C
TK-10
UO-31

Abb. 6

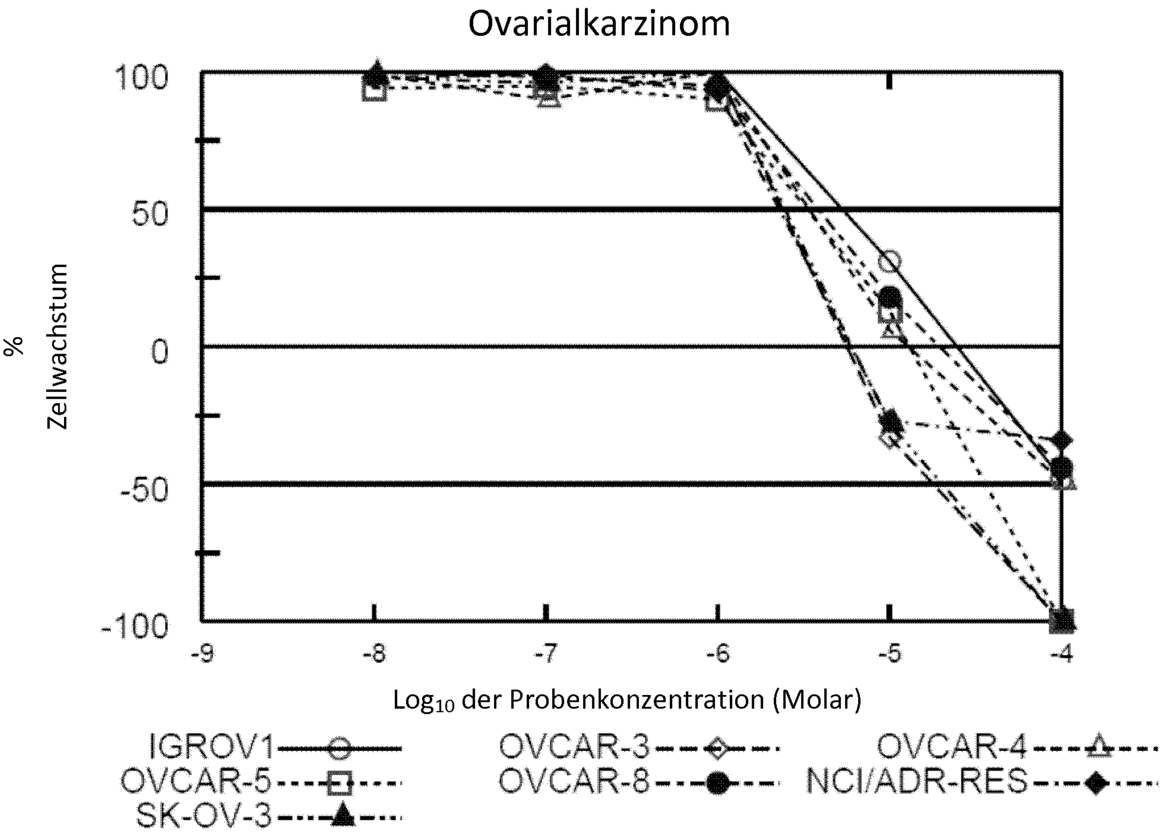
Ovarialkarzinom
100
50
0
-50
-100
% Zellwachstum
-9
-8
-7
-6
-5
-4
Log10 der Probenkonzentration (Molar)
IGROV1
OVCAR-3
OVCAR-4
OVCAR-5
OVCAR-8
NCI/ADR-RES
SK-OV-3

Abb. 7

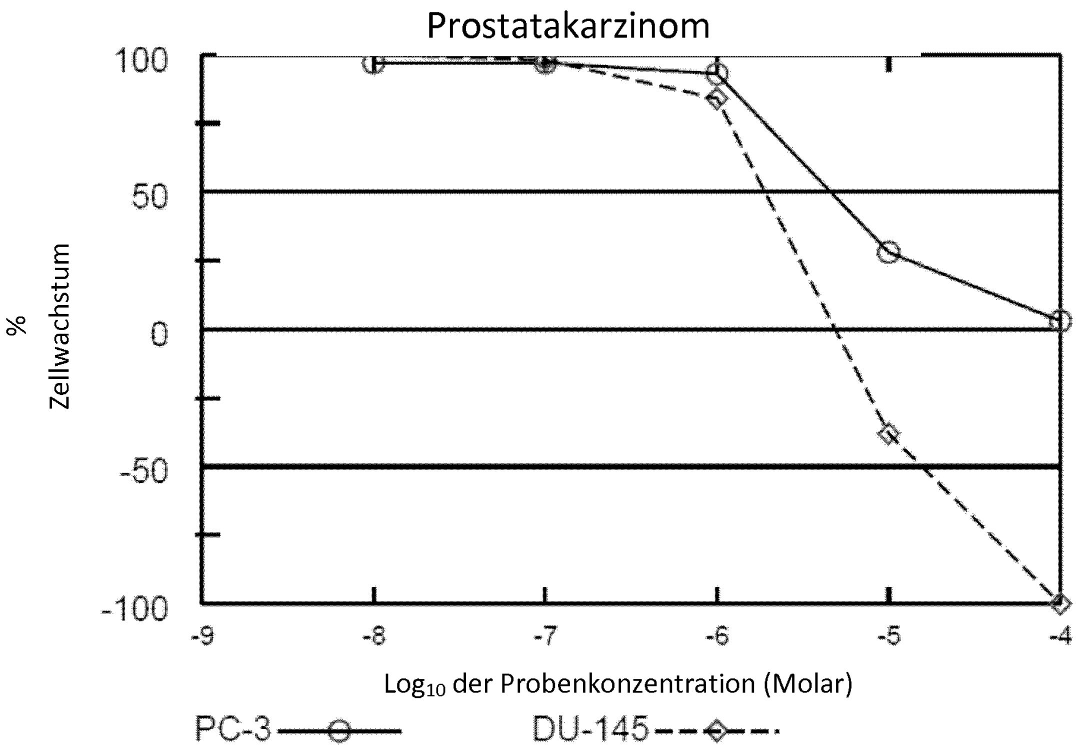
Prostatakarzinom
100
50
0
-50
-100
% Zellwachstum
-9
-8
-7
-6
-5
-4
Log10 der Probenkonzentration (Molar)
PC-3
DU-145

Abb. 8

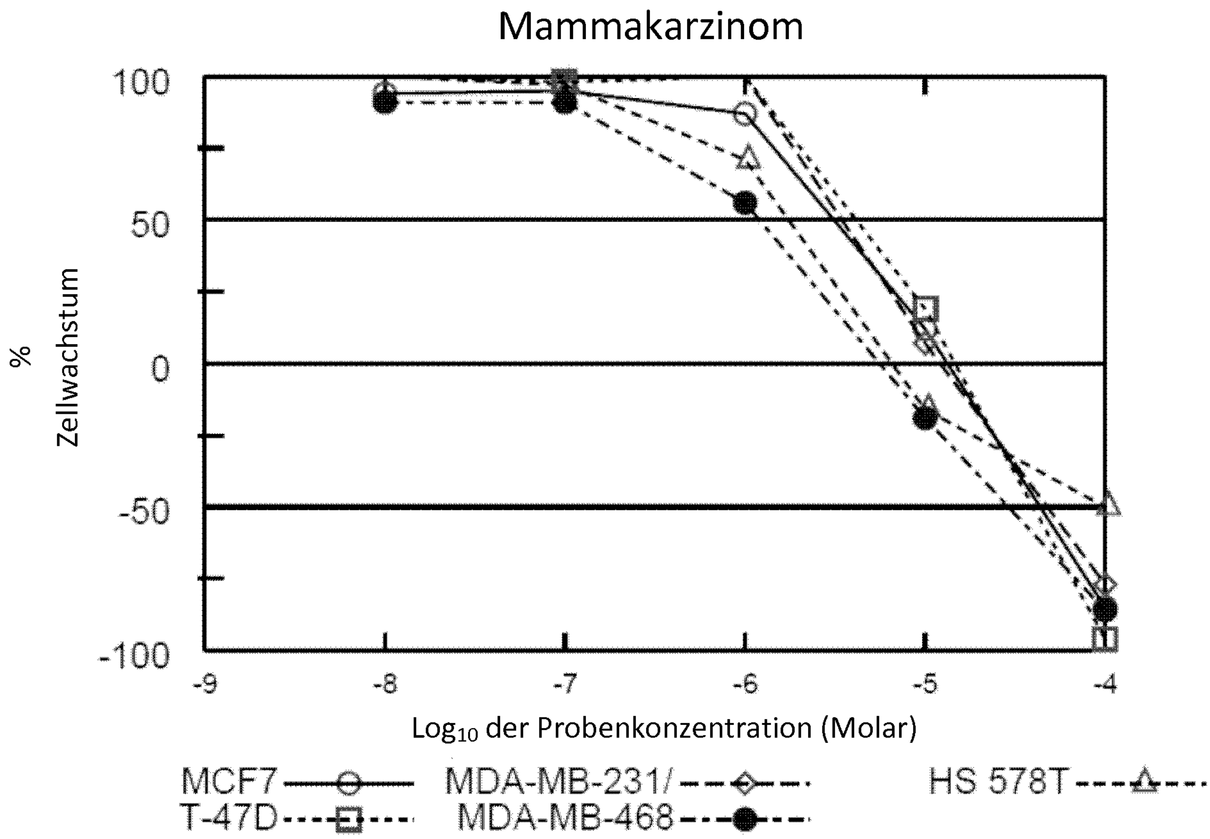
Mammakarzinom
100
50
0
-50
-100
% Zellwachstum
-9
-8
-7
-6
-5
-4
Log10 der Probenkonzentration (Molar)
MCF7
MDA-MB-231/
HS 578T
T-47D
MDA-MB-468

Abb. 9

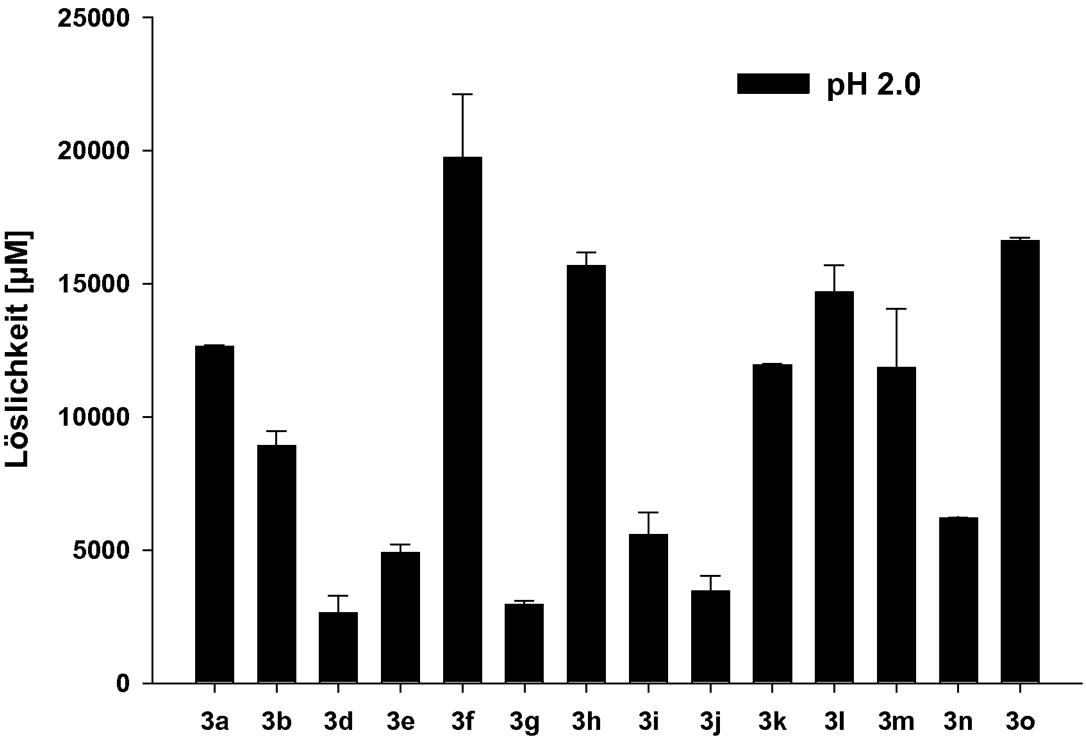
25000
20000
15000
10000
5000
0
Löslichkeit [µM]
pH 2.0
3a
3b
3d
3e
3f
3g
3h
3i
3j
3k
3l
3m
3n
3o

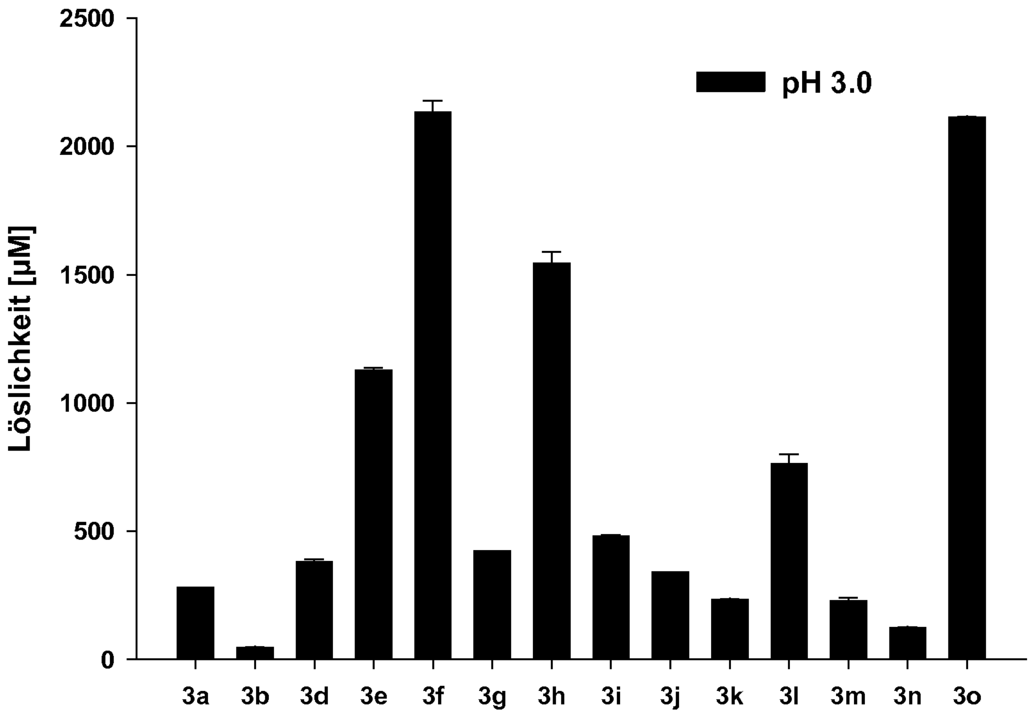
2500
2000
1500
1000
500
0
Löslichkeit [µM]
pH 3.0
3a
3b
3d
3e
3f
3g
3h
3i
3j
3k
3l
3m
3n
3o

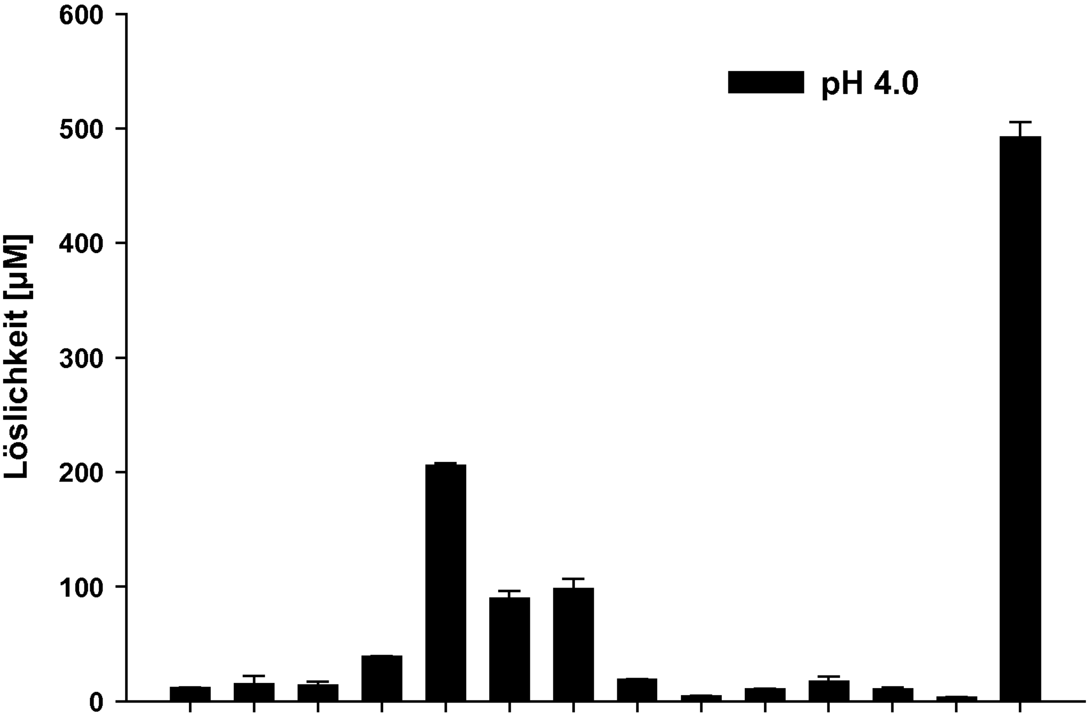
600
500
400
300
200
100
0
Löslichkeit [µM]
pH 4.0

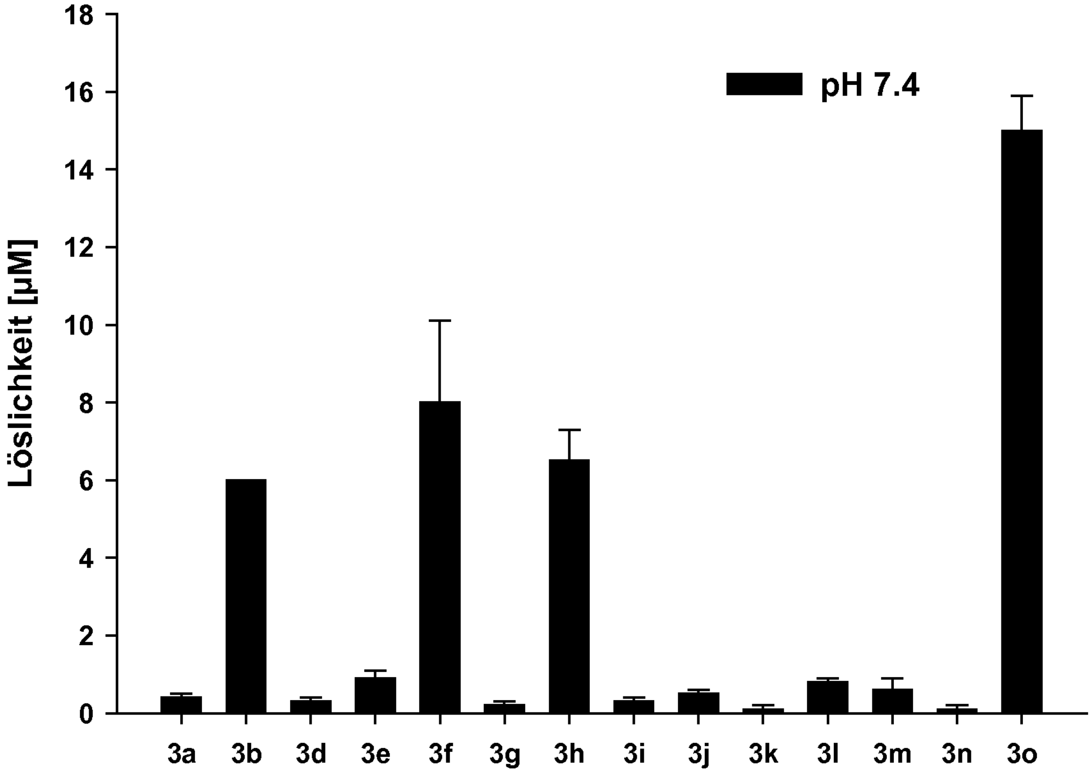
18
16
14
12
10
8
6
4
2
0
Löslichkeit [µM]
pH 7.4
3a
3b
3d
3e
3f
3g
3h
3i
3j
3k
3l
3m
3n
3o

Abb. 10 Fort.

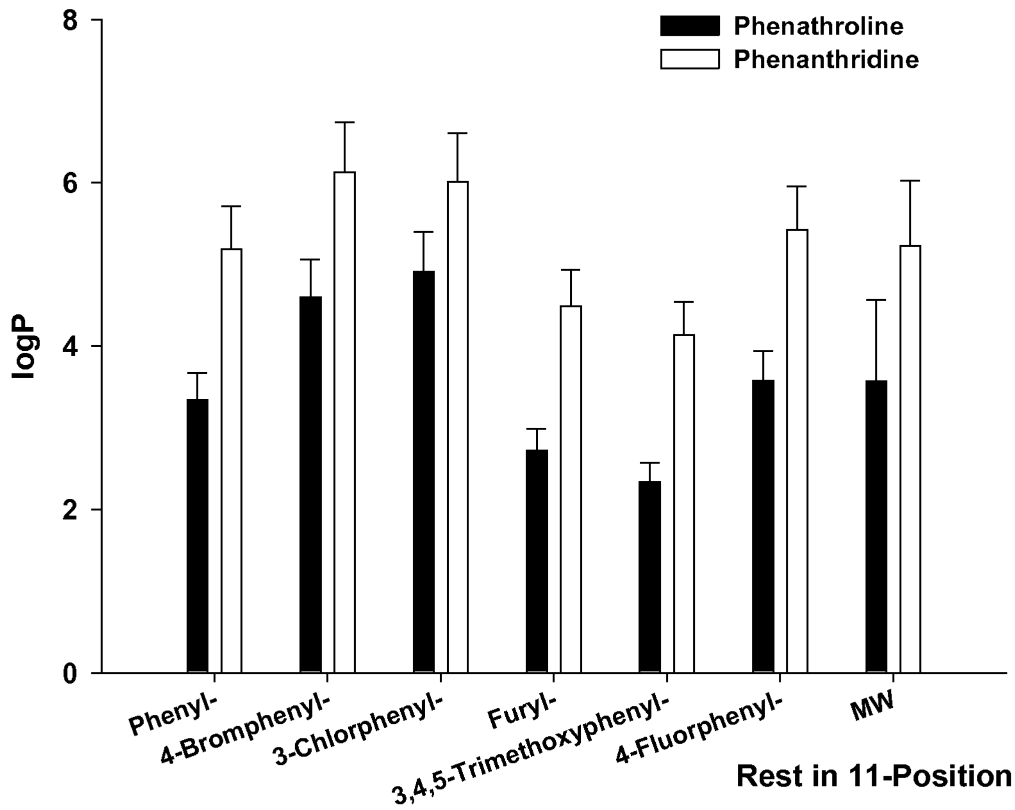
8
6
4
2
0
logP
Phenathroline
Phenanthridine
Phenyl-
4-Bromphenyl-
3-Chlorphenyl-
Furyl-
3,4,5-Trimethoxyphenyl-
4-Fluorphenyl-
MW
Rest in 11-Position

Abb. 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19538088 A1


### In der Beschreibung aufgeführte Nicht-Patentliteratur


- Organikum. Wiley-VCH, 2001
- **K. PETER ; C. VOLLHARDT.** Organische Chemie. 2000
- **RICHARD C. LAROCK.** *Comprehensive Organic Transformations,* 1989
- **GENES et al.** *Eu. J. Med. Chem.,* 2011, 2117-2131
- **CHERNG.** *Tetrahedron,* 2002, vol. 58, 4931-4935
- **K. PETER C. VOLLHARDT.** Organische Chemie. 2000
- **BOYD ; PAULL.** *Drug. Develop. Res.,* 1995, vol. 34, 91-109
- **POMMIER, Y.** *Nat. Rev. Cancer,* 2006, vol. 6, 789
- **POMMIER, Y.** *Chem. Rev.,* 2009, vol. 109, 2984
- **LIPINSKI, C. A. ; LOMBARDO, F. ; DOMINY, B. W. ; FEENEY, P. J.** *Adv. Drug. Deliv. Rev.,* 2001, vol. 46, 3-26